# EUROPEAN PATENT APPLICATION

(11) **EP 4 372 089 A1**
(43) Date of publication of application: **22.05.2024**
(21) Application number: 22842183.0
(22) Date of filing: 14.07.2022
(51) Int. Cl.: C12N 15/62, A61K 35/17, A61P 35/00, C07K 14/52, C07K 14/54, C07K 14/705, C07K 16/28, C07K 19/00, C12N 5/0783, C12N 5/10, C12N 15/19, C12N 15/24, C12N 15/63

(54) **CHIMERIC ANTIGEN RECEPTOR, CELL CAPABLE OF EXPRESSING SAID RECEPTOR, PHARMACEUTICAL COMPOSITION CONTAINING SAID CELL, METHOD FOR PRODUCING SAID CELL, AND POLYNUCLEOTIDE OR VECTOR WHICH CONTAINS NUCLEOTIDE SEQUENCE ENCODING SAID CHIMERIC ANTIGEN RECEPTOR**

(30) Priority: 16.07.2021 JP 2021118057
(71) Applicant: Noile-Immune Biotech Inc., Tokyo 105-0012 (JP)
(72) Inventor: TAMADA, Koji, Ube-shi, Yamaguchi 755-8505 (JP); SAKODA, Yukimi, Ube-shi, Yamaguchi 755-8505 (JP); ADACHI, Keishi, Ube-shi, Yamaguchi 755-8505 (JP)
(74) Representative: Dennemeyer & Associates S.A.
(86) International application number: PCT/JP2022/027736
(87) International publication number: WO 2023/286841

(57) **Abstract**

A chimeric antigen receptor includes a target antigen-binding domain, a transmembrane domain, and a signal transduction domain, wherein the transmembrane domain includes at its C-terminus a first polypeptide selected from the group consisting of
(1a) a polypeptide consisting of an amino acid sequence in which at least 3 amino acids from the C-terminus are deleted in the amino acid sequence set forth in SEQ ID NO: 1; (1b) a polypeptide consisting of an amino acid sequence having 85% or more sequence identity to the amino acid sequence of the polypeptide of (1a); and (1c) a polypeptide consisting of an amino acid sequence in which one or more amino acids are mutated in the amino acid sequence of the polypeptide of (1a), and a cell expressing the chimeric antigen receptor.

## Description

### Technical Field

The present disclosure relates to a chimeric antigen receptor, a cell expressing the receptor, a pharmaceutical composition including the cell, a method for producing the cell, and a polynucleotide or a vector including nucleotide sequence encoding the chimeric antigen receptor.

### Background Art

A chimeric antigen receptor (hereinafter, also referred to as "CAR") is an artificial chimeric protein in which a target antigen-binding region that recognizes a cell surface antigen of a cancer cell and a signal transduction region that induces activation of T cells are fused.

For example, a large amount of CAR-expressing T cells (hereinafter, also referred to as "CAR-T cells") can be produced by introducing a gene encoding a CAR into peripheral blood T cells (peripheral blood T lymphocytes). Such CAR-T cells have tumor reactivity and can bring injury of cancer cells without depending on an interaction with a major histocompatibility complex (MHC).

In cancer immunotherapy by administration of CAR-T cells, more specifically, a therapy in which T cells are collected from a patient, a gene encoding a CAR is introduced into the T cells and amplified, and the resulting T cells are transferred again to the patient, clinical trials are currently in progress worldwide, and results indicating effectiveness in, for example, a hematopoietic organ malignant tumor such as leukemia or lymphoma have been obtained.

Meanwhile, CAR-T cell therapy has been considered to be difficult to obtain an effect on a solid tumor. It is considered that this is because, for example, a survival ratio of CAR-T cells in a living body is low, accumulation of the CAR-T cells in a tumor site is low, and activity of the CAR-T cells is inhibited by an immunosuppressive factor or the like secreted by tumor cells.

As a method for solving such a problem, a method for introducing a nucleic acid encoding a T cell immune function promoting factor into a T cell together with a nucleic acid encoding a CAR has been reported. For example, JP 6761113 B2 discloses a CAR using a solid tumor antigen as a target antigen, and a CAR-T cell effective against a solid tumor.

### Summary of Invention

### Technical Problem

JP 6761113 B2 discloses a CAR-T cell that exhibits effectiveness against ganglioside GM2 as a target antigen. However, there is a constant demand for technology to enhance efficacy of CAR-T cell regardless of the target antigen.

A problem to be solved by an embodiment according to the present disclosure is to provide a chimeric antigen receptor having excellent cytotoxic activity.

A problem to be solved by another embodiment according to the present disclosure is to provide a cell expressing the chimeric antigen receptor, a polynucleotide or a vector including a nucleotide sequence encoding the chimeric antigen receptor, and a pharmaceutical composition including the cell.

### Solution to Problem

An embodiment of the present disclosure includes the following aspects.
<1> A chimeric antigen receptor includes a target antigen binding region, a transmembrane region, and a signal transduction region,
   wherein the transmembrane region includes at its C-terminus a first polypeptide selected from the group consisting of (1a) to (1c) below.
   (1a) a polypeptide consisting of an amino acid sequence in which at least 3 amino acids from the C-terminus are deleted in the amino acid sequence set forth in SEQ ID NO: 1;
   (1b) a polypeptide consisting of an amino acid sequence having 850 or more sequence identity to the amino acid sequence of the polypeptide of (1a); and (1c) a polypeptide consisting of an amino acid sequence in which one or more amino acids are mutated in the amino acid sequence of the polypeptide of (1a).
<2> The chimeric antigen receptor according to <1>, wherein the polypeptide of (1a) is a polypeptide consisting of an amino acid sequence in which 7 amino acids from the C-terminus of the amino acid sequence set forth in SEQ ID No: 1 are deleted.
<3> The chimeric antigen receptor according to <1> or <2>, wherein the transmembrane region further includes a second polypeptide selected from the group consisting of the following (2a) to (2c) on the N-terminal side of the first polypeptide:
   (2a) a polypeptide consisting of an amino acid sequence in which at least 3 amino acids from the N-terminus are deleted in the amino acid sequence set forth in SEQ ID No: 2;
   (2b) a polypeptide consisting of an amino acid sequence having 850 or more sequence identity to the amino acid sequence of the polypeptide of (2a); and
   (2c) a polypeptide consisting of an amino acid sequence in which one or more amino acids in the amino acid sequence of the polypeptide of (2a) are mutated.
<4> The chimeric antigen receptor according to <3>, wherein the polypeptide of (2a) is a polypeptide consisting of an amino acid sequence in which 10 amino acids from the N-terminal of the amino acid sequence set forth in SEQ ID No: 2 are deleted.
<5> The chimeric antigen receptor according to any one of <1> to <4>, wherein the signal transduction region is a T cell activation signal transduction region.
<6> A cell expressing the chimeric antigen receptor according to any one of <1> to <5>.
<7> The cell according to <6>, further expressing at least one of IL-7 and CCL19, preferably, further expressing at least one of exogenous IL-7 and exogenous CCL19.
<8> The cell according to <6> or <7>, wherein the cell is an immune cell.
<9> The cell according to <8>, wherein the immune cell is a T cell.
<10> A polynucleotide including a nucleotide sequence encoding the chimeric antigen receptor according to any one of <1> to <5>.
<11> A polynucleotide according to claim 10, wherein the chimeric antigen receptor further including a signal peptide at the N-terminus, the signal peptide is a polypeptide selected from the group consisting of the following (3a) to (3f):
   (3a) a polypeptide consisting of an amino acid sequence set forth in SEQ ID No: 3;
   (3b) a polypeptide consisting of an amino acid sequence having 850 or more sequence identity with the amino acid sequence set forth in SEQ ID NO: 3; and
   (3c) a polypeptide consisting of an amino acid sequence in which one or more amino acids in the amino acid sequence set forth in SEQ ID No: 3 are mutated;
   (3d) a polypeptide consisting of the amino acid sequence set forth in SEQ ID NO: 4;
   (3e) a polypeptide consisting of an amino acid sequence having 850 or more sequence identity with the amino acid sequence set forth in SEQ ID NO: 4; and
   (3f) a polypeptide consisting of an amino acid sequence in which one or more amino acids in the amino acid sequence set forth in SEQ ID No: 4 are mutated.
<12> The polynucleotide according to <11>, further including at least one of a nucleotide sequence encoding IL-7 and a nucleotide sequence encoding CCL19, preferably, further including at least one of a nucleotide sequence encoding IL-7 and a nucleotide sequence encoding CCL19 as exogenous nucleic acids.
<13> A vector including the polynucleotide of <11> or <12>.
<14> A method for producing a cell expressing a chimeric antigen receptor, the method including introducing the vector according to <13> into a cell.
<15> A pharmaceutical composition including the cell according to any one of <6> to <9>.
<16> The pharmaceutical composition according to <15>, which is a pharmaceutical composition for treating or preventing a tumor.

### Advantageous Effects of Invention

An embodiment of the present disclosure can provide a chimeric antigen receptor having excellent cytotoxic activity.

Another embodiment of the present disclosure can provide a cell expressing the chimeric antigen receptor, a polynucleotide or a vector including a nucleotide sequence encoding the chimeric antigen receptor, and a pharmaceutical composition including the cell.

### Brief Description of Drawings

Fig. 1A is a diagram illustrating an example of a result of measuring an expression level of an anti-EGFRviiiCAR by flow cytometry.
Fig. 1B is a diagram illustrating an example of a result of measuring an expression level of an anti-EGFRviiiCAR by flow cytometry.
Fig. 1C is a diagram illustrating an example of a result of measuring an expression level of an anti-EGFRviiiCAR by flow cytometry.
Fig. 1D is a diagram illustrating an example of a result of measuring an expression level of an anti-EGFRviiiCAR by flow cytometry.
Fig. 1E is a diagram illustrating an example of a result of measuring an expression level of an anti-EGFRviiiCAR by flow cytometry.
Fig. 1F is a diagram illustrating an example of a result of measuring an expression level of an anti-EGFRviiiCAR by flow cytometry.
Fig. 1G is a diagram illustrating an example of a result of measuring an expression level of an anti-EGFRviiiCAR by flow cytometry.
Fig. 1H is a diagram illustrating an example of a result of measuring an expression level of an anti-EGFRviiiCAR by flow cytometry.
Fig. 2A is a diagram illustrating an example of a result of measuring cytotoxic activity of EGFRviii CAR-T cells and activated T cells targeting EGFRviii non-expressing tumor cells by ATP assay.
Fig. 2B is a diagram illustrating an example of a result of measuring cytotoxic activity of EGFRviii CAR-T cells and activated T cells targeting EGFRviii expressing tumor cells by ATP assay.
Fig. 2C is a diagram illustrating an example of a result of measuring cytotoxic activity of EGFRviii CAR-T cells and activated T cells targeting EGFRviii non-expressing tumor cells by ATP assay.
Fig. 2D is a diagram illustrating an example of a result of measuring cytotoxic activity of EGFRviii CAR-T cells and activated T cells targeting EGFRviii expressing tumor cells by ATP assay.
Fig. 3A is a diagram illustrating an example of a result of measuring an expression level of an anti-EGFRviiiCAR by flow cytometry.
Fig. 3B is a diagram illustrating an example of a result of measuring an expression level of an anti-EGFRviiiCAR by flow cytometry.
Fig. 3C is a diagram illustrating an example of a result of measuring an expression level of an anti-EGFRviiiCAR by flow cytometry.
Fig. 4A is a diagram illustrating an example of a result of measuring cytotoxic activity of EGFRviii CAR-T cells and activated T cells targeting EGFRviii non-expressing tumor cells by ATP assay.
Fig. 4B is a diagram illustrating an example of a result of measuring cytotoxic activity of EGFRviii CAR-T cells and activated T cells targeting EGFRviii expressing tumor cells by ATP assay.
Fig. 5 is a diagram illustrating an example of a result of staining tumor cells with a PE-labeled anti-human EGFRviii antibody and performing flow cytometry analysis.
Fig. 6 is a diagram illustrating an example of a photograph of a back of a tumor model mouse to which tumor cells have been transplanted.
Fig. 7A is a graph illustrating an example of a result of a change in tumor volume of a tumor model mouse.
Fig. 7B is a graph illustrating an example of a result of a change in tumor volume of a tumor model mouse.
Fig. 7C is a graph illustrating an example of a result of a change in tumor volume of a tumor model mouse.

### Description of Embodiments

Hereinafter, contents of the present invention will be described in detail. Description of constitution requirements described below may be made based on a representative embodiment of the present invention, but the present invention is not limited to such an embodiment.

Note that, in the present specification, "to" indicating a numerical range is used to mean that numerical values described before and after the numerical range are included as a lower limit value and an upper limit value.

In a stepwisely described numerical range in the present disclosure, an upper limit value or a lower limit value described in one numerical range may be replaced with an upper limit value or a lower limit value of another stepwisely described numerical range. In addition, in a numerical range described in the present disclosure, an upper limit value or a lower limit value of the numerical range may be replaced with a value described in Examples.

In addition, in the present disclosure, "% by mass", "% by weight", and "Wt%" have the same meaning, and "parts by mass" and "parts by weight" have the same meaning.

Furthermore, in the present disclosure, a combination of two or more preferred aspects is a more preferred aspect.

A polypeptide, a polynucleotide, a vector, and a cell provided by the present disclosure can be in an isolated state. That is, a polypeptide, a polynucleotide, a vector, and a cell described in the present specification can be an isolated polypeptide, an isolated polynucleotide, an isolated vector, and an isolated cell.

### (Chimeric Antigen Receptor)

The chimeric antigen receptor (hereinafter referred to as "CAR") of the present disclosure includes a target antigen binding region, a transmembrane region, and a signal transduction region, wherein the transmembrane region includes at its C-terminus a first polypeptide selected from the group consisting of (1a) to (1c) below.
(1a) a polypeptide consisting of an amino acid sequence in which at least 3 amino acids from the C-terminus are deleted in the amino acid sequence set forth in SEQ ID No: 1;
(1b) a polypeptide consisting of an amino acid sequence having 850 or more sequence identity to the amino acid sequence of the polypeptide of (1a); and
(1c) a polypeptide consisting of an amino acid sequence in which one or more amino acids are mutated in the amino acid sequence of the polypeptide of (1a) above.

Note that, the above transmembrane domain does not include polypeptides including the 3 amino acids (Arg-Asn-Arg) from the C-terminus in SEQ ID NO: 1 at the C-terminus.

In the present specification, the "chimeric antigen receptor (CAR)" means an artificial chimeric protein obtained by fusing an antibody-derived portion that specifically recognizes an antigen (target antigen binding region), a transmembrane region, and a signal transduction region that induces activation of immune cells.

Note that the chimeric protein means a protein including a sequence derived from two or more different types of proteins.

The CAR of the present disclosure includes the first polypeptide selected from the group consisting of (1a) to (1c) at the C-terminus to enhance cytotoxic activity.

### <Transmembrane region>

The CAR according to the present disclosure includes a transmembrane region.

In the present specification, the "transmembrane region" means a region that penetrates a cell membrane and links an extracellular region and an intracellular region to each other when the CAR according to the present disclosure is expressed in a cell.

### <First polypeptide>

The transmembrane region included in the CAR of the present disclosure includes at its C-terminus a first polypeptide selected from the group consisting of (1a) to (1c). That is, the first polypeptide has the amino acid sequence of the transmembrane region of CD8.

### <<1a>>

The polypeptide of (1a) consists of an amino acid sequence in which at least 3 amino acids are deleted from the C-terminus in the amino acid sequence set forth in SEQ ID NO: 1. In (1a), the number of amino acid deletions from the amino acid sequence set forth in SEQ ID NO: 1 is at least 3. The maximum number of amino acid deletions may be, for example, 7. The number of amino acid deletions may be 3, 4, 5 or 6.

It is preferred that the polypeptide consisting of the amino acid sequence of SEQ ID NO: 1 in (1a) is a polypeptide consisting of an amino acid sequence in which 3 to 7 amino acids are deleted from the C-terminus in the amino acid sequence set forth in SEQ ID NO: 1. It is more preferred that the polypeptide consisting of an amino acid sequence in which 4 to 7 amino acids are deleted from the C-terminus in the amino acid sequence set forth in SEQ ID NO: 1. It is further preferred that the polypeptide consisting of an amino acid sequence in which 5 to 7 amino acids are deleted from the C-terminus in the amino acid sequence set forth in SEQ ID NO: 1. It is particularly preferred that the polypeptide consisting of an amino acid sequence in which 6 or 7 amino acids are deleted from the C-terminus in the amino acid sequence set forth in SEQ ID NO: 1. it is most preferred that the polypeptide consisting of an amino acid sequence in which 7 amino acids are deleted from the C-terminus in the amino acid sequence set forth in SEQ ID NO: 1.

### «Sequence identity»

In (1b) above, the sequence identity to the amino acid sequence of the polypeptide in (1a) is not particularly limited as long as it is 85% or more, but 90% or more is preferred, 950 or more is more preferred, and 96%, 97%, 98%, or 99% or more is even preferred.

Note that, the polypeptide of (1b) does not include the polypeptide including the 3 amino acids (Arg-Asn-Arg) from the C-terminus in SEQ ID NO: 1 at the C-terminus.

Note that the sequence identity between amino acid sequences is determined by juxtaposing two amino acid sequences with a gap in a portion corresponding to insertion and deletion such that the largest number of corresponding amino acids match each other, and determining a ratio of matched amino acids to the entire amino acid sequence excluding the gap in the obtained alignment.

The sequence identity between amino acid sequences can be determined using various types of homology search software known in the present technical field. For example, a value of the sequence identity of the amino acid sequence can be obtained by calculation based on an alignment obtained by known homology search software BLASTP.

The sequence identity between the following amino acid sequences is also determined in the same way.

### <<Number of amino acid mutations >>

In (1c), the number of amino acid mutations (number of mutated amino acid residues) from the amino acid sequence of the polypeptide in (1a) may be one or more. The "plurality" may be from 2 to 5, for example, and may be 2, 3, 4, or 5, for example. The "plurality" is preferably from 2 to 4, more preferably from 2 to 3, and even more preferably from 2 to 5. The "mutations" may be deletions, substitutions, additions, and insertions, or a combination thereof. Note that polypeptides in (1c) do not include polypeptides that include the 3 amino acids (Arg-Asn-Arg) from the C-terminus in SEQ ID NO: 1 at the C-terminus.

### -Extracellular hinge region-

The above transmembrane region may include an extracellular hinge region as part of the transmembrane region. As used herein, the term "extracellular hinge region" refers to the region located at the N-terminus of the transmembrane domain and linked to the extracellular target antigen binding region. The protein including the extracellular hinge region may be derived from a natural protein or artificially designed. The extracellular hinge region may also be a mutant of the extracellular hinge region derived from a natural protein.

An organism from which a protein in the extracellular hinge region is derived is not particularly limited, and may be a human, a mouse, a rat, a hamster, a rabbit, or a monkey, but a human is preferable. Note that amino acid sequences of these proteins constituting the extracellular hinge region are available from a known sequence database such as GenBank.

Extracellular hinge regions included as part of the transmembrane region include, for example, extracellular hinge regions of CD8, CD28, CD4, etc. The hinge region of immunoglobulins (e.g., IgG4, etc.) may also be used as part of the transmembrane region. When the extracellular hinge region is included as part of the transmembrane region, the extracellular hinge region of CD8 is preferred as the extracellular hinge region. As the amino acid sequence of the extracellular hinge region, the amino acid sequence of the extracellular hinge region of CD8 is suitably mentioned, for example, the amino acid sequences set forth in SEQ ID NO: 2 and SEQ ID NO: 5.

### <Second polypeptide>

From the viewpoint of superior cytotoxic activity, it is preferred that the transmembrane region further includes an extracellular hinge region on the N-terminal side of the first polypeptide. For example, it is more preferred that the transmembrane region further includes the second polypeptide selected from the group consisting of the following (2a) to (2c) as an extracellular hinge region. That is, the second polypeptide is an extracellular hinge region.

(2a) a polypeptide consisting of an amino acid sequence in which at least 3 amino acids from the N-terminus are deleted in the amino acid sequence set forth in SEQ ID NO: 2;
(2b) a polypeptide consisting of an amino acid sequence having 850 or more sequence identity to the amino acid sequence of the polypeptide of (2a); and
(2c) a polypeptide consisting of an amino acid sequence in which one or more amino acids are mutated in the amino acid sequence of the polypeptide of (2a).

In (2a), the number of amino acid deletions from the N-terminus of the amino acid sequence set forth in SEQ ID NO: 2 is preferably at least 3. The upper limit of the number of amino acid deletions can be, for example, 10. The lower limit of the number of amino acid deletions is not limited and may be 3, 4, 5, 6, 7, 8 or 9.

The polypeptide of (2a) is preferably a polypeptide consisting of an amino acid sequence in which 3 to 10 amino acids are deleted from the N-terminus of the amino acid sequence set forth in SEQ ID NO: 2, more preferably a polypeptide consisting of an amino acid sequence in which 5 to 10 amino acids are deleted from the N-terminus of the amino acid sequence set forth in SEQ ID NO: 2, further preferably a polypeptide consisting of an amino acid sequence in which 7 to 10 amino acids are deleted from the N-terminus of the amino acid sequence set forth in SEQ ID NO: 2, especially preferably a polypeptide consisting of an amino acid sequence in which 9 or 10 amino acids are deleted from the N-terminus of the amino acid sequence set forth in SEQ ID NO: 2, most preferably a polypeptide consisting of an amino acid sequence in which 10 amino acids are deleted from the N-terminus of the amino acid sequence set forth in SEQ ID NO: 2.

In the amino acid sequence (2b) of the second polypeptide, the sequence identity to the amino acid sequence of the polypeptide in (2a) is not particularly limited as long as it is 85% or more, but 90% or more is preferred, 950 or more is more preferred, 96%, 97%, 98% or 99% or more is further preferred.

Note that polypeptides in (2b) do not include polypeptides that include the 3 amino acids (Phe-Val-Pro) from the N-terminus in SEQ ID NO 2 at the C-terminus.

The sequence identity between the amino acid sequences is obtained in the same way as the method for determining the sequence identity between the amino acid sequences in the amino acid sequence of the first polypeptide above.

In the amino acid sequence (2c) of the second polypeptide above, "plurality" may be from 2 to 5, for example, it can be 2, 3, 4, or 5. The "plurality" is preferably from 2 to 4, more preferably from 2 to 3, and even more preferably from 2 to 5. The "mutations" may be deletions, substitutions, additions, and insertions, or a combination thereof.

Note that polypeptides in (2c) do not include polypeptides including the 3 amino acids (Phe-Val-Pro) from the N-terminus in SEQ ID NO: 2 at the C-terminus.

### <Signal transduction region>

The CAR according to the present disclosure includes a signal transduction region. The signal transduction region only needs to be a region that can transmit a signal into a cell expressing the CAR according to the present disclosure when the target antigen-binding site binds to a target antigen.

In the present specification, the "signal transduction region" means a region that is located in a cell and transmits a signal into the cell when the CAR according to the present disclosure is expressed in the cell.

Note that the above cell is synonymous with the CAR-expressing cell described below, and the same is true for the preferred form.

In a T cell, when a major histocompatibility complex (MHC)-peptide complex binds to a T cell receptor (TCR), a T cell activation signal is transmitted into the cell via a TCR-CD3 complex, and various phosphorylation signals are induced (primary signal transduction). For example, in a T cell transfected with the chimeric antigen receptor (CAR) gene (also referred to as "CAR-T cell"), a primary signal can be transmitted to the T cell without depending on an interaction with MHC by binding of an antigen to a target antigen-binding site.

In addition, it is known that a costimulatory molecule expressed on a cell surface of a T cell (for example, CD28 or 4-1BB described later) transmits a costimulatory signal into a cell by binding to a ligand specific to each costimulatory molecule expressed on a cell surface of an antigen-presenting cell, and assists activation of the T cell (secondary signal transduction). In the CAR-T cell, by incorporating such a costimulatory molecule into the CAR, a secondary signal can be transmitted to the T cell when a target antigen-binding site binds to an antigen.

In the present specification, the "T cell activation signal transduction" includes both the primary signal transduction and the secondary signal transduction. In addition, the "T cell activation signal transduction region" means an intracellular region of a protein involved in at least one of the primary signal transduction and the secondary signal transduction, involved in the signal transduction.

The signal transduction region is not particularly limited as long as it is a cell activation signal transduction region of a protein involved in activation signal transduction of a cell.

For example, it is known that an immunoreceptor tyrosine-based activation motif (ITAM) is involved in the primary signal transduction. Therefore, examples of the signal transduction region include a cell activation signal transduction region of a protein having an ITAM.

Examples of the protein having an ITAM include CD3ζ, FcRγ, FcRβ, CD3γ, CD3δ, CD3ε, CD5, CD22, CD79a, CD79b, and CD66d. The signal transduction region including an ITAM is a preferred example of the cell activation signal transduction region used in the CAR according to the present disclosure. More preferred examples of the signal transduction region include a cell activation signal transduction region such as CD3ζ.

In addition, a costimulatory molecule is involved in the secondary signal transduction as described above. Therefore, examples of the signal transduction region also include a signal transduction region of a costimulatory molecule.

Examples of the costimulatory molecule include CD2, CD4, CD5, CD8, CD27, CD28, OX40 (CD134), 4-1BB (CD137), ICOS, CD154, herpesvirus entry mediator (HVEM), GITR, and FcReceptor-associated γchain. Cell activation signal transduction regions of these proteins are also preferred examples of the cell activation signal transduction region used in the CAR according to the present disclosure.

More preferred examples thereof include cell activation signal transduction regions of CD28 and 4-1BB.

An organism from which a protein in the signal transduction region is derived is not particularly limited, and may be a human, a mouse, a rat, a hamster, a rabbit, or a monkey, but a human is preferable. Note that amino acid sequences of these proteins are available from a known sequence database such as GenBank.

Examples of an amino acid sequence of human CD3ζ include an amino acid sequence registered as GenBank number: NM_000734.3, and examples of an amino acid sequence of the signal transduction region include the amino acid sequence set forth in SEQ ID NO: 7.

Examples of an amino acid sequence of human CD28 include an amino acid sequence registered as GenBank number: NM_006139.2, and examples of an amino acid sequence of the signal transduction region include the amino acid sequence set forth in SEQ ID NO: 8.

Examples of an amino acid sequence of human 4-1BB include an amino acid sequence registered as GenBank number: NM_001561.5, and examples of an amino acid sequence of the signal transduction region include the amino acid sequence set forth in SEQ ID NO: 9.

The signal transduction region may be a variant of a signal transduction region derived from a natural protein as described above. Examples of the variant of an activation signal transduction region derived from a natural protein include the following:
(4a) A polypeptide consisting of an amino acid sequence having 70% or more of sequence identity (homology) with an amino acid sequence of a signal transduction region derived from a natural protein (for example, SEQ ID NO: 7 to 9), and having cell activation signal transduction ability; and
(4b) A polypeptide consisting of an amino acid sequence having one or a plurality of amino acids mutated in an amino acid sequence of a signal transduction region derived from a natural protein (for example, SEQ ID NO: 7 to 9), and having cell activation signal transduction ability.

In the amino acid sequence (4a) of the signal transduction region, the sequence identity is not particularly limited as long as it is 70% or more, but is preferably 80% or more, more preferably 85% or more, still more preferably 90% or more, and particularly preferably 95% or more.

In the above amino acid sequence (4b), "a plurality" may be, for example, 2 to 30, is preferably 2 to 20, more preferably 2 to 10, and still more preferably 2 to 5 in a case where a protein involved in the primary signal transduction is used. In addition, "a plurality" may be, for example, 2 to 15, and is preferably 2 to 10, more preferably 2 to 5, and still more preferably 2 or 3 in a case where a costimulatory molecule is used.

The "mutation" may be any of deletion, substitution, addition, and insertion, and may be a combination thereof.

The number of signal transduction regions included in the CAR according to the present disclosure is not limited to one, and a plurality of signal transduction regions may be included. In this case, the plurality of signal transduction regions may be the same signal transduction region or different signal transduction regions.

The CAR according to the present disclosure preferably includes two or more signal transduction regions. In this case, the signal transduction region included in the CAR according to the present disclosure is preferably a combination of a signal transduction region involved in the primary signal transduction and a signal transduction region involved in the secondary signal transduction.

Specific examples thereof include a combination of cell activation signal transduction regions of CD3 ζ and CD28, a combination of cell activation signal transduction regions of CD3ζ and 4-1BB, and a combination of cell activation signal transduction regions of CD3ζ, CD28, and 4-1BB.

Note that in a case where a signal transduction region involved in the primary signal transduction and a signal transduction region involved in the secondary signal transduction are combined, the signal transduction region involved in the primary signal transduction is preferably arranged on a C-terminus side.

In the above specific example, a T cell activation signal transduction region of CD3ζ is preferably arranged on a C-terminus side of a T cell activation signal transduction region of CD28 or 4-1BB. In a case where both CD28 and 4-1BB are used, CD28 and 4-1BB may be arranged in any order, but for example, CD28 and 4-1BB may be arranged in this order from an N-terminus side.

As an example of a signal transduction region, when using CD3ζ in combination with the 4-1BB cell activation signal transduction region, the signal transduction region preferably include, starting from the N-terminus, the 4-1BB co-stimulation signal region and the CD3ζ signal region in succession.

In a case where only one T cell activation signal transduction region is used, a T cell activation signal transduction region involved in the primary signal transduction is preferably used, and a T cell activation signal transduction region of CD3ζ is more preferably used.

### <Target antigen-binding region>

The CAR according to the present disclosure includes a target antigen-binding region. The "target antigen-binding region" means a region that binds to the target antigen and is located outside the cell when the CAR of the present disclosure is expressed in a cell. The above-mentioned cells are synonymous with the cells expressing the CARs described below, and the same applies to the preferred form.

For example, a CAR expressed in a T cell into which a chimeric antigen receptor (CAR) gene has been introduced (CAR-T cell) migrates to a cell membrane, and to be in state in which a target antigen-binding region located outside the cell and a T cell activation signal transduction region located in the cell are linked to each other via a transmembrane region penetrating the cell membrane. When the CAR-T cell comes into contact with a cell having a target antigen as a membrane antigen, the target antigen-binding region binds to the target antigen, whereby a T cell activation signal is transmitted from the T cell activation signal transduction region into the T cell, and the T cell is activated.

The target antigen binding region is not restricted as long as the peptide has sufficient affinity to the target antigen, and can be designed according to the purpose. For example, it may include the target antigen binding site of a T cell receptor or the target antigen binding site of a single chain antibody (scFv: single chain variable fragment).

The scFv is a polypeptide in which a heavy chain variable region (VH region) of an antibody and a light chain variable region (VL region) of the antibody are linked to each other with a peptide linker.

In the scFv, the order in which the VH region and the VL region are linked to each other with a peptide linker is not particularly limited, and may be the order of the VH region, the peptide linker, and the VL region, or the order of the VL region, the peptide linker, and the VH region from an N-terminus side.

In an embodiment of the present disclosure, the scFv preferably includes a light chain variable region of the antibody, a peptide linker linking a heavy chain variable region and the light chain variable region to each other, and the heavy chain variable region sequentially from an N-terminus.

In the scFv, the peptide linker linking the VH region and the VL region to each other is not particularly limited, and it is only required to use those generally used for the scFv.

Examples of the peptide linker include linker 15 (SEQ ID NO: 15) and linker 25 (SEQ ID NO: 16), but are not limited thereto.

The target antigen binding region may also be a domain binder single (e.g., from camelids); an artificial binder single (e.g., Darpin); or a single chain derived from a T cell receptor.

The target antigen binding region may be an antibody or an antigen-binding fragment.

The above antibody or the antigen-binding fragment is not particularly limited as long as it is peptide with sufficient affinity to the target antigen, and examples thereof include small molecule antibodies such as a single chain variable fragment(scFv), Fab, diabody, ScDb, scFv₂-Fc, IgG-scFv, scFv-HSA-scFv, Fab-scFv-Fc, Fab-scFv, and scFv-Fc.

There are no restrictions as to target antigens, the target antigen may be any biomolecule that is specifically expressed on a cancer cell and its progenitor cells, any biomolecule that is newly expressed as a result of cell transformation to cancer, or any biomolecule whose expression level is increased in cancer cells compared to normal cells.

Examples of the target antigen include, for example, CD20, EGFR, FITC, CD19, CD22, CD33, PSMA, GD2, EGFRviii, ROR1, c-Met, HER2, CEA, mesothelin, GM2, CD5, CD7, CD10, CD13, CD30, CD34 CD38, CD41, CD44, CD52, CD74, CD123, CD133, CD138, CD160, CD171, CA-125, MUC16, MUC1, CS1 (CD319), IL-13Ra2, BCMA, LewisY, IgGkappa chain, Folate receptor-alpha, PSCA, EpCAM, WT1, MART-1, NY-ESO-1, MAGE - A1, MAGE-A3, MAGE-A4, Glypican-3, KIF20A, Survivin, AFP-1, gp100, PAP -10, PAP-5, TRP2-1, SART-1, VEGFR1, VEGFR2, NEIL3, MPHOSPH1, DEPDC1, FOXM1, CDH3, TTK, TOMM34, URLC10, KOC1, UBE2T, TOPK, ECT2, NKG2D, P1A, 5T4, B7-H6, EphA2, ErbB2, FAP, FR-a, TARP, DLL3, PRSS21, Claudin18.2, Claudin1 8, CAIX, L1-CAM, FAP-α, CTAG1B, NCAM, ALK, vimentin, carbonic anhydrase IX, G250, A33, etc.

The CAR of the present disclosure may also be a bispecific antibody with target antigen binding regions that bind to two different target antigens.

The "antigen-binding region" of an antibody refers to the region of the antibody that is involved in binding to the antigen, specifically, the region including the complementarity determining region (CDR).

The antigen binding region of the antibody includes at least one CDR of the antibody. In a preferred form, the antigen-binding region of the antibody includes all six CDRs of the antibody.

Note that CDR can be determined by any definition known as a definition of CDR, and for example, a definition by Kabat, Chothia, AbM, contact, IMGT, Aho, or Mrtin (Enhanced Chothia) can be used.

### [Other region]

The CAR according to the present disclosure may include a region other than the target antigen binding region, the transmembrane region and the signal transduction region (hereinafter, also referred to as "other region"). Examples of the other region include a spacer region, and a signal peptide.

### -Spacer region-

In the CAR according to the present disclosure, regions such as the target antigen-binding region, the transmembrane region, and the signal transduction region may be linked to each other via a spacer region.

In the present specification, the "spacer region" refers to a short peptide linking two functional regions (domains) of a protein to each other. The spacer region is not particularly limited, and it is only required to use those generally used for producing a chimeric protein.

The length of the spacer region may be 1 to 100 amino acids, and preferably 10 to 50 amino acids. Examples of the spacer region include a glycine-serine continuous sequence.

### -Signal peptide-

The CAR according to the present disclosure may include a signal peptide.

In the present specification, the "signal peptide" refers to a peptide that indicates localization of a membrane protein or a secreted protein. The signal peptide is generally a peptide including about 5 to 60 amino acids present at an N-terminus of a membrane protein, and a signal peptide has been removed in a mature protein in which localization has been completed.

The signal peptide included in the CAR according to the present disclosure is preferably a signal peptide that indicates localization to a cell membrane, and preferably a signal peptide of a membrane protein.

Examples of the signal peptide include signal peptides such as an α chain and a β chain of a T cell receptor, CD3ζ, CD28, CD3ε, CD45, CD4, CD5, CD8, CD9, CD16, CD22, CD33, CD37, CD64, CD80, CD86, CD134, CD137, ICOS, CD154, GITR, an immunoglobulin heavy chain, and an immunoglobulin light chain.

Specific examples of an amino acid sequence of the signal peptide include the amino acid sequences set forth in SEQ ID NOs: 3 and 4.

It is also preferred that the above signal peptide is a polypeptide selected from the group consisting of the following (3a) to (3f).
(3a) a polypeptide consisting of an amino acid sequence set forth in SEQ ID NO: 3;
(3b) a polypeptide consisting of an amino acid sequence having 850 or more sequence identity with the amino acid sequence set forth in SEQ ID NO: 3; and
(3c) a polypeptide consisting of an amino acid sequence in which one or more amino acids are mutated in the amino acid sequence set forth in SEQ ID NO: 3;
(3d) polypeptide consisting of the amino acid sequence set forth in SEQ ID NO: 4;
(3e) a polypeptide consisting of an amino acid sequence having 850 or more sequence identity with the amino acid sequence set forth in SEQ ID NO: 4; and
(3f) a polypeptide consisting of an amino acid sequence in which one or more amino acids are mutated in the amino acid sequence set forth in SEQ ID NO: 4.

In (3b) above, the sequence identity to the amino acid sequence of the polypeptide in (3a) is not particularly limited as long as it is 85% or more, but it is preferred to be 90% or more, 950 or more, 960 or more, 970 or more, 980 or more, or 99% or more.

In (3e) above, the sequence identity to the amino acid sequence of the polypeptide in (3d) is not particularly limited as long as it is 85% or more, but it is preferred to be 90% or more, 950 or more, 960 or more, 970 or more, 980 or more, or 99% or more.

In (3c) above, the number of amino acid variations from the amino acid sequence of the polypeptide in (3a) may be one or more. The "plurality" may be from 2 to 5, for example, and may be 2, 3, 4, or 5, for example. The "plurality" is preferably from 2 to 4, more preferably from 2 to 3, and even more preferably from 2 to 5. The "mutations" may be any of deletion, substitution, addition, and insertion, and may be a combination thereof.

In (3f) above, the number of amino acid variations from the amino acid sequence of the polypeptide in (3d) may be one or more. The "plurality" may be from 2 to 5, for example, and may be 2, 3, 4, or 5, for example. The "plurality" is preferably from 2 to 4, more preferably from 2 to 3, and even more preferably from 2 to 5. The "mutations" may be any of deletion, substitution, addition, and insertion, and may be a combination thereof.

In the CAR according to the present disclosure, the above regions are preferably arranged in the order of the target antigen-binding region, the transmembrane region, and the signal transduction region from an N-terminus. These regions may be directly linked to each other, or may be linked to each other via another region, a spacer sequence, or the like.

In a case where the CAR according to the present disclosure includes a signal peptide, the signal peptide is preferably arranged at an N-terminus of the polypeptide.

The example of CAR of the present disclosure include CAR including the target antigen binding region including the scFv of anti-EGFRviii antibody, the transmembrane region of CD8 (or a mutant thereof), the cell activation signal transduction region of CD28 (or a mutant thereof), the T cell activation signal transduction region of 4-1BB (or a mutant thereof) and the cell activation signal transduction region of CD3ζ (or a mutant thereof).

### (Cell)

A cell according to the present disclosure is preferably a cell expressing the CAR according to the present disclosure.

The Cell expressing CAR is not restricted as long as it is activated when its target antigen-binding region binds to the target antigen on the tumor cell surface and can destroy tumor cell by releasing cytotoxic granules or cytokines.

The cell according to the present disclosure is preferably a mammalian cell. The mammalian cell may be, for example, a human cell or a cell of a non-human mammal such as a mouse, a rat, a cow, a sheep, a horse, a dog, a pig, or a monkey. Among these, the mammalian cell is more preferably a human cell.

The type of cell is not particularly limited, and a cell that can reach a cancer tissue through a blood vessel can be widely used. Example thereof include a cell collected from blood, bone marrow fluid, spleen, thymus, lymph node, or the like, an immune cell that infiltrates a cancer tissue such as a primary tumor, a metastatic tumor, or cancerous ascites, and a mesenchymal stem cell.

In the present disclosure, the "immune cell" means a cell that performs an immune function in a living body(Preferably cell isolated from living organisms). The immune cell may also be referred to as an immunocompetent cell. The immune cell is preferably a cell separated from a living body. Examples of the immune cell include a lymphocyte such as a T cell, a natural killer cell (NK cell), or a B cell, an antigen-presenting cell such as a monocyte, a macrophage, or a dendritic cell, and a granulocyte such as a neutrophil, an eosinophil, a basophil, or a mast cell.

More specifically, a peripheral blood mononuclear cell and the like separated from peripheral blood can be suitably used.

Among cells included in the peripheral blood mononuclear cell, an effector cell is preferable, and as the effector cell, for example, a T cell and a precursor cell thereof are used. The type of T cell is not particularly limited, and may be any T cell such as an αβT cell, a γδT cell, a CD8 positive T cell, a cytotoxic T cell, a CD4 positive T cell, a helper T cell, a memory T cell, a naive T cell, a tumor-infiltrating T cell, or a natural killer T cell.

Among these, the T cell is more preferably a CD8 positive T cell or a cytotoxic T cell.

### <<IL-7 and CCL19>>

The cell according to the present disclosure preferably further expresses at least one of interleukin (IL)-7 (IL-7) and chemokine (C-C motif) ligand 19 (CCL19) in addition to the CAR.

The cell according to the present disclosure is preferably a cell expressing (i) the CAR according to the present disclosure and (ii) at least one of IL-7 and CCL19, and more preferably a cell expressing the CAR according to the present disclosure, IL-7, and CCL19. The IL-7 and/or CCL19 are preferably exogenous, that is, expressed from an exogenous nucleic acid.

IL-7 is an essential cytokine for T cell survival, and is produced by a non-hematopoietic cell such as a stromal cell of bone marrow, thymus, and lymphatic organs/tissues, but production in the T cell is hardly observed.

Meanwhile, CCL19 is produced mainly from a dendritic cell and a macrophage in the lymph node, and has a function of inducing migration of a T cell, a B cell, and a mature dendritic cell via a CC chemokine (C-C motif) receptor 7 (CCR7) which is a receptor of CCL19.

An organism from which each of IL-7 and CCL19 is derived is not particularly limited, and may be a human, a mouse, a rat, a hamster, a rabbit, or a monkey, but a human is preferable. Note that amino acid sequences of these proteins are available from a known sequence database such as GenBank.

Examples of an amino acid sequence of human IL-7 include an amino acid sequence registered as GenBank number: NM_000880.3 (SEQ ID NO: 10). Examples of an amino acid sequence of human CCL19 include an amino acid sequence registered as GenBank number: NM_006274.2 (SEQ ID NO: 11).

Note that IL-7 and CCL19 each include a signal peptide, and the signal peptide is removed in a mature protein.

For example, in the amino acid sequence of human IL-7 set forth in SEQ ID NO: 10, a sequence of positions 1 to 25 corresponds to a signal peptide. In addition, for example, in the amino acid sequence of human CCL19 set forth in SEQ ID NO: 11, a sequence of positions 1 to 21 corresponds to a signal peptide.

In addition, IL-7 and CCL19 may be each a variant of a natural protein as described above. Examples of the variant of IL-7 include the following:
(6a) A polypeptide consisting of an amino acid sequence having 70% or more of sequence identity (homology) with an amino acid sequence of natural IL-7 (for example, SEQ ID NO: 10), and having a T cell immune function promoting function; and
(6b) A polypeptide consisting of an amino acid sequence having one or a plurality of amino acids mutated in an amino acid sequence of natural IL-7 (for example, SEQ ID NO: 10), and having a T cell immune function promoting function.

Examples of a variant of CCL19 include the following.
(7a) A polypeptide consisting of an amino acid sequence having 70% or more of sequence identity (homology) with an amino acid sequence of natural CCL19 (for example, SEQ ID NO: 11), and having a T cell immune function promoting function.
(7b) A polypeptide consisting of an amino acid sequence having one or a plurality of amino acids mutated in an amino acid sequence of natural CCL19 (for example, SEQ ID NO: 11), and having a T cell immune function promoting function.

Note that the "T cell immune function promoting function" means a function of maintaining and/or promoting survival, proliferation, cytotoxic activity, migration activity, infiltration activity into tumor tissue, and the like of a T cell.

In the above (6a) and (7a), the sequence identity is not particularly limited as long as it is 70% or more, but is preferably 80% or more, more preferably 85% or more, still more preferably 90% or more, and particularly preferably 95% or more.

In addition, in the above (6b) and (7b), "a plurality" may be, for example, 2 to 30, and is preferably 2 to 20, more preferably 2 to 10, and still more preferably 2 to 5.

The "mutation" may be any of deletion, substitution, addition, and insertion, and may be a combination thereof.

In addition, the variants of IL-7 and CCL19 may be obtained by changing signal peptides of these proteins to other signal peptides, or may be obtained by removing the signal peptides. Preferably, the variants of IL-7 and CCL19 each include a signal peptide of a secreted protein and are secreted to the outside of a cell.

In a case where the cell according to the present disclosure is an isolated T cell, by expressing at least one selected from the group consisting of IL-7 and CCL19, preferably from an exogenous nucleic acid, together with a CAR, the T cell immune function is promoted, and the cell can be a cell excellent in cytotoxic activity against a tumor antigen-expressing cell, infiltration into a tumor tissue, or viability in a tumor microenvironment.

The cell according to the present disclosure, preferably the immune cell, may further express another immune function control factor such as IL-15, CCL 21, IL-2, IL-4, IL-12, IL-13, IL-17, IL-18, IP-10, Interferon-γ, MIP-1alpha, GM-CSF, M-CSF, TGF-beta, or TNF-alpha, and the other immune control factor is preferably an immune function control factor other than IL-12. Note that these other immune control factors may be expressed from endogenous nucleic acids encoding the respective factors, or may be expressed from exogenous nucleic acids encoding the respective factors.

In addition, the cell according to the present disclosure may express a suicide gene in addition to the CAR. Since the cell according to the present disclosure expresses a suicide gene, apoptosis can be induced in the cell according to the present disclosure as necessary.

The suicide gene is not particularly limited, and a known suicide gene can be used. Examples of the suicide gene include a thymidine kinase (HSV-TK) gene of herpes simplex virus and an inducible caspase 9 gene. A cell expressing HSV-TK can induce cell death by coexisting ganciclovir.

In addition, a cell expressing inducible caspase 9 can induce cell death by coexisting a chemical induction of dimerization (CID) such as AP1903.

An amino acid sequence of the suicide gene is available from a known sequence database such as GenBank. In addition, a sequence such as a vector including a commercially available suicide gene can also be used.

The cell according to the present disclosure preferably includes a CAR.

The cell according to the present disclosure is preferably a cell including a CAR and at least one selected from the group consisting of IL-7 and CCL19, more preferably a cell including a CAR, IL-7, and CCL19, and may be a cell including a CAR, IL-7, CCL19, and a suicide gene. Each of the IL-7 and CCL19 is preferably expressed from an exogenous nucleic acid (such as a vector introduced from the outside of a cell) encoding the IL-7 and CCL19 themselves.

The cell according to the present disclosure can be obtained by introducing a polynucleotide or a vector including a nucleotide sequence encoding a CAR described later into a cell.

### (Polynucleotide)

The polynucleotide according to the present disclosure preferably includes a nucleotide sequence encoding the CAR.

### <Nucleotide sequence>

Example of the nucleotide sequence encoding the target antigen binding region can be designed according to the target antigen. If the target antigen binding region binds to a tumor antigen, the nucleotide sequence encoding the scFv of the antibody against the tumor antigen is one of example of the nucleotide sequence.

The nucleotide sequence encoding the target antigen-binding region is preferably codon-optimized according to the species of a cell to be introduced, and is preferably optimized to a human codon in a case of introduction into a human cell.

In addition, nucleotide sequences encoding the transmembrane region and the signal transduction region are available from a known sequence database such as GenBank.

For example, in a case where a transmembrane region of human CD8 is used as the transmembrane region, examples of a nucleotide sequence encoding human CD8 include an amino acid sequence registered as GenBank No.: NM_001768.6, and examples of a nucleotide sequence encoding the transmembrane region include the nucleotide sequence set forth in SEQ ID NO: 17.

In addition, for example, in a case where T cell activation signal transduction regions of human CD3ζ, human CD28, and human 4-1BB are used as the T cell activation signal transduction region, examples of nucleotide sequences encoding human CD3ζ, human CD28, and human 4-1BB include amino acid sequences registered as GenBank numbers: NM_000734.3, NM_006139.2, and NM_001561.5, respectively, and examples of nucleotide sequences encoding the T cell activation signal transduction regions of human CD3ζ, human CD28, and human 4-1BB include the nucleotide sequence set forth in SEQ ID NOs: 18 to 20, respectively.

The nucleotide sequence encoding each of the above regions is not limited to a known one, and may be any sequence as long as it is a nucleotide sequence encoding each of the above regions. Due to degeneracy of a genetic code, there is a plurality of codons corresponding to one amino acid. Therefore, there are many nucleotide sequences encoding the same amino acid sequence. The nucleotide sequence encoding each of the above regions may be any of a plurality of nucleotide sequences generated by degeneracy of a genetic code as long as it encodes these regions.

The nucleotide sequence encoding each of the above regions is preferably codon-optimized according to the species of a cell to be introduced, and is preferably optimized to a human codon in a case of introduction into a human cell.

In addition, the nucleotide sequence encoding each of the regions may encode a variant of each of the regions derived from a natural protein. The variant of each of the regions is as described above.

In the nucleotide sequence encoding each of the regions of the CAR according to the present disclosure, a nucleotide sequence encoding the target antigen-binding region, a nucleotide sequence encoding the transmembrane region, and a nucleotide sequence encoding the signal transduction region are preferably arranged in this order from a 5' side.

In a case where the signal peptide, the extracellular hinge region, and the like are used, preferably, a nucleotide sequence encoding the signal peptide is arranged on a 5' side of the nucleotide sequence encoding the target antigen-binding region, and a nucleotide sequence encoding the extracellular hinge region is arranged between the nucleotide sequence encoding the target antigen-binding region and the nucleotide sequence encoding the transmembrane region.

The nucleotide sequences encoding these regions may be directly linked to each other, or may be linked to each other via a nucleotide sequence encoding a spacer region.

The spacer region is as described above.

The polynucleotide according to the present disclosure is obtained by linking polynucleotides each including a nucleotide sequence encoding each of the regions of the CAR to each other directly or via a spacer sequence. The polynucleotide encoding each of the regions of the polypeptide may be obtained by chemical synthesis by a known method based on a nucleotide sequence of each of the regions.

Alternatively, the polynucleotide encoding each of the regions of the polypeptide may be obtained by using DNA extracted from a T cell or the like and cDNA obtained by reverse transcription of RNA extracted from a T cell or the like as a template, and amplifying a polynucleotide encoding each of the regions, for example, by an isothermal amplification method such as a PCR method, a LAMP method, or a TRC method.

The polynucleotide encoding each of the regions thus obtained may be modified by substitution, deletion, addition, or insertion within a range in which the function of each of the regions after translation is not lost.

In addition to the nucleotide sequence encoding an CAR, the polynucleotide according to the present disclosure may include a control sequence such as a promoter, an enhancer, a poly A addition signal, or a terminator, a nucleotide sequence encoding another protein, and the like.

Examples of the other protein include IL-7 and CCL19. Nucleotide sequences encoding these proteins are available from a known sequence database such as GenBank.

For example, in a case where human IL-7 is used, examples of a nucleotide sequence encoding human IL-7 include an amino acid sequence registered as GenBank number: NM_002190.2 (SEQ ID NO: 21). In a case where human CCL19 is used, examples of a nucleotide sequence encoding human CCL19 include an amino acid sequence registered as GenBank number: NM_006274.2 (SEQ ID NO: 22).

In addition, the nucleotide sequences encoding these proteins are not limited to known ones, and may be any sequences as long as they are nucleotide sequences encoding these proteins, and may be any of a plurality of nucleotide sequences generated by degeneracy of a genetic code. The nucleotide sequences encoding these proteins are preferably codon-optimized according to the species of a cell to be introduced, and are preferably optimized to a human codon in a case of introduction into a human cell.

In addition, the nucleotide sequences encoding these proteins may encode variants of natural IL-7 and natural CCL19. These variants are as described above.

In addition, examples of the other protein include a suicide gene. The suicide gene is as described in the above.

A nucleotide sequence encoding the suicide gene is available from a known sequence database such as GenBank. In addition, a sequence such as a vector including a commercially available suicide gene can also be used.

In a case where the polynucleotide according to the present disclosure includes a nucleotide sequence encoding another protein, a nucleotide sequence encoding a self-cleaving peptide such as a 2A peptide, an internal ribozyme entry site (IRES) sequence, or the like may be interposed between the nucleotide sequence encoding the CAR and the nucleotide sequence encoding the other protein.

In addition, in a case where there are two or more other proteins, a self-cleaving peptide, IRES, or the like may be interposed between the other proteins. By interposing these sequences, a plurality of proteins can be independently expressed from one promoter.

Examples of the 2A peptide include 2A peptides such as picornavirus, rotavirus, insect virus, aphthovirus, and trypanosoma virus.

As a specific example, an amino acid sequence of the 2A peptide (F2A) of picornavirus is represented by SEQ ID NO: 14. A nucleotide sequence encoding the 2A peptide is preferably codon-optimized according to the species of a cell to be introduced, and is preferably optimized to a human codon in a case of introduction into a human cell. An amino acid sequence at a cleavage position of the 2A peptide is represented by SEQ ID NO: 13, and a protein is translated in a form of being cleaved between proline at a C-terminus and glycine adjacent to proline toward an N-terminus side.

In addition, the polynucleotide according to the present disclosure may have a control sequence such as a promoter, an enhancer, a poly A addition signal, or a terminator for each of protein coding sequences of the nucleotide sequence encoding the CAR and the nucleotide sequence encoding another protein. In addition, some protein coding sequences may each independently have a control sequence, and other protein coding sequences may be linked thereto via the 2A peptide, IRES, or the like to have a common control sequence.

### (Vector)

The vector according to the present disclosure preferably includes a nucleotide sequence encoding the CAR. The polynucleotide may be in a form of a vector.

The type of vector is not particularly limited, and examples thereof include a generally used expression vector.

The vector may be linear or circular, may be a non-virus vector such as a plasmid, may be a virus vector, or may be a vector based on a transposon.

Examples of the vector include a virus vector, a plasmid vector, an episomal vector, and an artificial chromosome vector.

Examples of the virus vector include a Sendai virus vector, a retrovirus (including lentivirus) vector, an adenovirus vector, an adeno-associated virus vector, a herpesvirus vector, a vaccinia virus vector, a poxvirus vector, a poliovirus vector, a sindbis virus vector, a rhabdovirus vector, a paramyxovirus vector, and an orthomyxovirus vector.

Examples of the plasmid vector include plasmid vectors for animal cell expression, such as pA1-11, pXT1, pRc/CMV, pRc/RSV, and pcDNAI/Neo.

The episomal vector is a vector capable of autonomously replicating extrachromosomally. Examples of the episomal vector include a vector including a sequence necessary for autonomous replication derived from an EV virus (EBV), Simian virus 40 (SV40), or the like as a vector element. Specific examples of the vector element required for autonomous replication include a replication initiation point and a gene encoding a protein that binds to the replication initiation point and controls replication.

Examples thereof include a replication initiation point oriP and an EBNA-1 gene in EBV, and a replication initiation point ori and an SV40LT gene in SV40.

Examples of the artificial chromosome vector include a yeast artificial chromosome (YAC) vector, a bacterial artificial chromosome (BAC) vector, and a P1-derived artificial chromosome (PAC) vector.

Suitable examples of the vector according to the present disclosure include a virus vector, and more suitable examples thereof include a retrovirus vector.

Examples of the retrovirus vector include a pMSGV1 vector (Tamada K et al., Clin Cancer Res 18: 6436-6445 (2012)) and a pMSCV vector (manufactured by Takara Bio Inc.). By using the retrovirus vector, a gene in the vector is incorporated into a genome of a host cell, and can be stably expressed in the host cell for a long period of time.

The vector according to the present disclosure may include a nucleotide sequence encoding a replication initiation point and a protein that binds to the replication initiation point and controls replication, a nucleotide sequence encoding a marker gene such as a drug-resistant gene or a reporter gene, and the like.

The nucleotide sequence encoding the CAR according to the present disclosure is preferably arranged in a vector so as to be expressed under control of an appropriate promoter. In a case where the vector includes a nucleotide sequence encoding another protein, the nucleotide sequences is also preferably arranged in the vector so as to be expressed under control of an appropriate promoter.

Examples of the promoter include an SRα promoter, an SV40 early promoter, a retroviral LTR, a cytomegalovirus (CMV) promoter, a Rous sarcoma virus (RSV) promoter, a herpes simplex virus thymidine kinase (HSV-TK) promoter, an EF1α promoter, a metallothionein promoter, and a heat shock promoter.

In addition, an enhancer of an IE gene of human CMV may be used together with the promoter. Examples thereof include a CAG promoter (including a cytomegalovirus enhancer, a chicken β-actin promoter, and a poly-A signal site of a β-globin gene). In addition, a nucleotide sequence encoding a self-cleaving peptide or IRES may be arranged between protein coding sequences, and transcription may be performed under control of a common promoter.

The vector according to the present disclosure preferably further includes at least one of a nucleotide sequence encoding IL-7 and a nucleotide sequence encoding CCL19 in addition to the nucleotide sequence encoding the CAR, and more preferably further includes a nucleotide sequence encoding IL-7 and a nucleotide sequence encoding CCL19 in addition to the nucleotide sequence encoding the CAR.

The vector preferably includes a nucleotide sequence encoding a CAR functionally linked to an appropriate promoter, and more preferably, the nucleotide sequence encoding the CAR, the nucleotide sequence encoding IL-7, and the nucleotide sequence encoding CCL19 each include a nucleotide sequence encoding a self-cleaving peptide or a nucleotide sequence linked via IRES, and the nucleotide sequence is functionally linked to an appropriate promoter.

### <<Combination of nucleotide sequences included in vector>>

A combination of nucleotide sequences included in the vector is not particularly limited, and a nucleotide sequence encoding the CAR, a nucleotide sequence encoding IL-7, and a nucleotide sequence encoding CCL19 may be carried on separate vectors and introduced into a cell. Two or more nucleotide sequences selected from these nucleotide sequences may be combined, carried on the same vector, and introduced into a cell.

For example, in a case where the CAR according to the present disclosure, IL-7, and CCL19 are expressed, nucleotide sequences encoding these may be carried on separate vectors and introduced into a cell, two types of vectors, that is, a vector carrying a nucleotide sequence encoding the CAR according to the present disclosure and a nucleotide sequence encoding IL-7 and a vector carrying a nucleotide sequence encoding CCL-19 may be introduced into a cell, two types of vectors, that is, a vector carrying a nucleotide sequence encoding the CAR according to the present disclosure and a nucleotide sequence encoding CCL19 and a vector carrying a nucleotide sequence encoding IL-7 may be introduced into a cell, two types of vectors, that is, a vector carrying a nucleotide sequence encoding the CAR according to the present disclosure and a nucleotide sequence encoding IL-7 and a vector carrying a nucleotide sequence encoding the CAR according to the present disclosure and a nucleotide sequence encoding CCL19 may be introduced into a cell, or one type of vector, that is a vector carrying a nucleotide sequence encoding the CAR according to the present disclosure, a nucleotide sequence encoding IL-7, and a nucleotide sequence encoding CCL19 may be introduced into a cell.

Note that, in the present specification, "functionally linked to a promoter" means being linked downstream of the promoter so as to be expressed under control of the promoter. In the above example, the arrangement order of the nucleotide sequence encoding a CAR, the nucleotide sequence encoding IL-7, and the nucleotide sequence encoding CCL19 is not particularly limited, and may be any arrangement order.

### (Method for producing cell expressing CAR)

A method for producing a cell expressing the CAR according to the present disclosure (hereinafter, also simply referred to as "method for producing a cell expressing a CAR") preferably includes introducing the CAR into a cell. In addition, the method for producing a cell expressing the CAR according to the present disclosure preferably includes introducing the CAR or vector into a cell.

A method for introducing the polynucleotide or vector into a cell is not particularly limited, and a known method can be used. Examples thereof include a virus infection method, a lipofection method, a microinjection method, a calcium phosphate method, a DEAE-dextran method, an electroporation method, a method using a transposon, and a particle gun method.

In a case where the vector used in the method for producing a cell expressing a CAR is a retrovirus vector, an appropriate packaging cell may be selected based on an LTR sequence and a packaging signal sequence included in the vector, and a retroviral particle may be prepared using the selected packaging cell.

Examples of the packaging cell include PG13, PA317, GP+E-86, GP+envAm-12, and Psi-Crip. In addition, a 293 cell or a 293T cell having a high transfection efficiency can also be used as the packaging cell.

Since various retrovirus vectors and packaging cells that can be used for packaging the vectors are widely commercially available, these commercially available products may be used. For example, a GP2-293 cell (manufactured by Takara Bio Inc.), a Plat-GP cell (manufactured by Cosmo Bio Inc.), a PG13 cell (ATCCCRL-10686), and a PA317 cell (ATCCCRL-9078) can be used, and a commercially available kit such as Retrovirus packaging Kit Eco (manufactured by Takara Bio Inc.) may be used.

In a case where another exogenous protein such as IL-7, CCL19, or a suicide gene is expressed in a cell expressing the CAR according to the present disclosure, a nucleotide sequence encoding each of these other proteins may be included in a vector including a nucleotide sequence encoding the CAR according to the present disclosure, or may be included in another vector. In a case where a nucleotide sequence encoding another protein is included in another vector, the vector can be introduced into a cell simultaneously with or separately from a vector including a nucleotide sequence encoding the CAR according to the present disclosure.

In addition, the cell expressing the CAR according to the present disclosure may be produced by incorporating a polynucleotide including a nucleotide sequence encoding the CAR according to the present disclosure into a genome of the cell so as to be able to be expressed under control of an appropriate promoter using a known gene editing technique or the like.

Examples of the gene editing technique include a technique using an endonuclease such as zinc finger nuclease, transcription activation-like effector nuclease (TALEN), clustered regularly interspaced short palindromic repeat (CRISPR)-Cas system, or pentatricopeptide repeat (PPR) .

Similarly, in a case where another exogenous protein is expressed in a cell expressing the CAR according to the present disclosure, a polynucleotide including a nucleotide sequence encoding another exogenous protein may be incorporated into a genome of the cell so as to be able to be expressed under control of an appropriate promoter using a gene editing technique or the like. Examples thereof include: a method for incorporating a polynucleotide including a nucleotide sequence encoding the CAR according to the present disclosure (or another protein) functionally linked to an appropriate promoter into a non-coding region or the like of a cell genome.; and a method for incorporating a polynucleotide including a nucleotide sequence encoding the CAR according to the present disclosure (or another protein) downstream of an endogenous promoter of a cell genome.

Examples of the endogenous promoter include promoters of TCRα and TCRβ.

### -Method for confirming expression-

After a polynucleotide or a vector including a nucleotide sequence encoding the CAR according to the present disclosure is introduced into a cell, expression of the CAR in the cell can be confirmed by a known method such as flow cytometry, RT-PCR, Northern blotting, Western blotting, ELISA, or fluorescent immunostaining. In addition, expression of another exogenous protein such as IL-7 or CCL19 can be similarly confirmed by a known method.

### (Pharmaceutical composition)

A pharmaceutical composition according to the present disclosure preferably includes the above cell.
the cell expressing the CAR according to the present disclosure exhibits specific cytotoxic activity against a cell expressing the target antigen. Therefore, the cell expressing the CAR can be used for treating or preventing a tumor in which the cell expressing the target antigen is involved.

The tumor may be generated in any of a bone tissue, a cartilage tissue, an adipose tissue, a muscle tissue, a vascular tissue, and a hematopoietic tissue. Examples of the tumor include: cancers such as glioma, melanoma, malignant mesothelioma, lung cancer, pancreatic cancer, head and neck cancer, liver cancer, uterine cancer, bladder cancer, biliary tract cancer, esophageal cancer, testicular tumor, thyroid cancer, brain tumor, prostate cancer, large bowel cancer, renal cancer, ovarian cancer, breast cancer, adenocarcinoma, squamous cell cancer, adenosquamous cancer, anaplastic cancer, large cell cancer, small cell cancer, skin cancer, vaginal cancer, neck cancer, spleen cancer, tracheal cancer, bronchial cancer, small intestine cancer, stomach cancer, gallbladder cancer, and testicular cancer; sarcomas such as osteosarcoma, chondrosarcoma, Ewing's sarcoma, malignant hemangioendothelioma, malignant schwannoma, and soft tissue sarcoma; blastomas such as neuroblastoma, hepatoblastoma, medulloblastoma, nephroblastoma, pancreatic blastoma, pleuropulmonary blastoma, and retinoblastoma; a germ cell tumor; and hematologic cancers such as lymphoma, leukemia, and myeloma, but are not limited thereto.

In particular, the pharmaceutical composition according to the present disclosure is suitable as a pharmaceutical composition for treating or preventing a tumor expressing target antigens that can bind to the target antigen binding sites included in the CARs of the present disclosure.

The method of confirming the expression of the target antigen that can bind to the target antigen binding site included in the CAR of the present disclosure in a tumor can be set according to the type, characteristics, or the like of the target antigen.

When the target antigen is, for example, a tumor antigen, examples of the known method for confirming expression of the tumor antigen include flow cytometry, ELISA, immunostaining, and fluorescent immunostaining.

A cell expressing either or both of IL-7 and CCL19 (preferably expressed from an exogenous nucleic acid) in addition to the CAR according to the present disclosure can exhibit strong cytotoxic activity even against a solid tumor. Therefore, a pharmaceutical composition including the cell expressing the CAR according to the present disclosure and either or both of IL-7 and CCL19 can be particularly suitably used for a solid tumor. Therefore, the pharmaceutical composition for treating or preventing a solid tumor, the pharmaceutical composition including the cell expressing the CAR according to the present disclosure and either or both of IL-7 and CCL19, is a suitable example of the pharmaceutical composition according to the present disclosure.

The "solid tumor" means a tumor other than hematologic cancer generated from a hematopoietic tissue, and includes epithelial cell cancer and non-epithelial cell cancer.

The pharmaceutical composition according to the present disclosure may include, in addition to the cell expressing the CAR according to the present disclosure, another component such as a pharmaceutically acceptable carrier.

Examples of the other component include a pharmaceutically acceptable carrier, a T cell activation factor such as cytokine, an immunostimulant, an immune checkpoint inhibitor, a cell expressing another CAR, and an anti-inflammatory agent, but are not limited thereto. Examples of the pharmaceutically acceptable carrier include a cell culture medium, physiological saline, a phosphate buffer, and citrate buffer.

The pharmaceutical composition according to the present disclosure can be administered by a known method, but preferably can be administered to a patient by injection or infusion. An administration route is preferably intravenous administration, but is not limited thereto, and administration may be performed, for example, by injection into a tumor.

The pharmaceutical composition according to the present disclosure can include a therapeutically effective amount of the cell expressing the CAR according to the present disclosure.

In the present specification, the "therapeutically effective amount" means an amount of a drug effective for treating or preventing a disease. The therapeutically effective amount may vary depending on a disease condition, an age, a sex, a weight, and the like of an administration target. In the pharmaceutical composition according to the present disclosure, the therapeutically effective amount of the cell expressing the CAR is, for example, an amount in which the cell expressing the CAR can suppress proliferation of a tumor, and is preferably an amount that leads to reduction of a tumor.

An administration amount and an administration interval of the pharmaceutical composition according to the present disclosure can be appropriately selected according to an age, a sex, a weight, and the like of an administration target, a type, a progression degree, a symptom, and the like of a disease, an administration method, and the like.

As the administration amount, a therapeutically effective amount can be administered, and for example, in one administration, the number of administered cells is 1 × 10⁴ to 1 × 10¹⁰, preferably 1 × 10⁵ to 1 × 10⁹, and more preferably 5 × 10⁶ to 5 × 10⁸.

The administration interval of the pharmaceutical composition according to the present disclosure can be, for example, every week, every 10 days to 30 days, every month, every three months to six months, or every year. In addition, since the cell expressing the CAR according to the present disclosure can autonomously proliferate in a body of an administration target, administration can be performed only once. In addition, the number of cells expressing the CAR in the body may be monitored after administration, and a time of administration may be determined according to a result thereof.

In addition, the pharmaceutical composition according to the present disclosure can be used in combination with another anticancer agent. Examples of the other anticancer agent include an alkylating agent such as cyclophosphamide, an antimetabolite such as pentostatin, a molecular targeting agent such as rituximab, a kinase inhibitor such as imatinib, a proteasome inhibitor such as bortezomib, a calcineurin inhibitor such as cyclosporine, an anticancer antibiotic such as idarubicin, a plant alkaloid such as irinotecan, a platinum preparation such as cisplatin, a hormone therapy agent such as tamoxifen, and an immunomodulatory agent such as nivolumab or pembrolizumab, but are not limited thereto.

In addition, the administration target of the pharmaceutical composition according to the present disclosure is not particularly limited, and may be a human, a monkey, a dog, or the like.

In another aspect, the present disclosure provides: 1) use of a cell expressing the CAR according to the present disclosure in manufacture of a pharmaceutical composition for treating or preventing a tumor; 2) a method for treating or preventing a tumor, including administering a cell expressing the CAR according to the present disclosure to a target (for example, a patient suffering from a tumor expressing a tumor antigen targeted by the CAR or a patient with surgical resection of a tumor); 3) a cell expressing the CAR according to the present disclosure to be used for treating or preventing a tumor; and 4) use of a cell expressing the CAR according to the present disclosure for treating or preventing a tumor.

Furthermore, in another aspect, the present disclosure also provides a kit for producing a cell expressing the CAR according to the present disclosure, including the vector according to the present disclosure. The kit is not particularly limited as long as it includes the vector according to the present disclosure, and may include an instruction for producing a cell expressing the CAR according to the present disclosure, a reagent used for introducing the vector into a cell, and the like.

### Examples

Hereinafter, the present disclosure will be described with reference to Examples, but the present disclosure is not limited to the following Examples.

### (Example 1) Production of CAR-T cells (1)

### <Design of CAR construct>

In the following example, anti-EGFRviiiCAR-T cells expressing IL-7, CCL19, and HSV-TK were produced with anti-EGFRviiiscFV disclosed in the amino acid sequence set forth in SEQ ID NO: 6 as the target antigen-binding region (with an additional signal peptide of SEQ ID NO: 3 on the N-terminal side). The sequence of the transmembrane region was examined by producing the anti-EGFRviiiCAR-T cells. The method for producing CAR-T cells followed the method described in WO 2017/159736 A.

The CAR constructs were constructed sequentially including anti-EGFRviiiscFv, human CD8 transmembrane region, and 4-1BB and CD3ζ intracellular signaling regions from the N-terminal side. The above CAR construct is a so-called second-generation CAR construct. F2A, which is a 2A peptide, was added to a C-terminus of the construct, and human IL-7-F2A-human CCL19-F2A-HSV-TK was further added downstream thereof.

The anti-EGFRviiiscFv was an scFv having the amino acid sequence set forth in SEQ ID NO: 6. The scFv includes, from the N-terminus, a VL region, a linker 25 (SEQ ID NO: 16), and a VH region.

In this study, the two types of human CD8 transmembrane regions, CD8 Long and CD8 Short, whose sequences are shown below were used.

CD8 Long (SEQ ID NO 12) 
FVPVFLPAKPTTTPAPRPPTPAPTIASQPLSLRPEACRPAAGGAVHTRGLDFACDIYIW

APLAGTCGVLLLSLVITLYCNHRNR
CD8 Short (SEQ ID NO 23)

TTTPAPRPPTPAPTIASQPLSLRPEACRPAAGGAVHTRGLDFACDIYIWAPLAGTCGVL

LLSLVITL

The following two types of signal sequences were used as the N-terminus of the anti-EGFRviii scFv.
IgG SS (SEQ ID NO 3)
   MDWTWRILFLVAAATGAHS
CD8 SS (SEQ ID NO 4)
   MALPVTALLLPLALLLHAARP

Other sequences are shown in Table 1 below.

**[Table 1]**

| Sequence No. | Sequence name | Amino acid sequence |
|---|---|---|
| 9 | 4-1 BB_A_aa | RFSVVKRGRKKLLYIFKQPFMRPVQTTQEEDGCSCRFPEEEEGG CE |
| 7 | CD3z_A_aa | LRVKFSRSADAPAYQQGQNQLYNELNLGRREEYDVLDKRRGR DPEMGGKPRRKNPQEGLYNELQKDKMAEAYSEIGMKGERRRG KGHDGLYQGLSTATKDTYDALHMQALPPR |
| 10 | IL-7 human_aa | MFHVSFRYIFGLPPLILVLLPVASSDCDIEGKDGKQYESVLMVSI DQLLDSMKEIGSNCLNNEFNFFKRHICDANKEGMFLFRAARKL RQFLKMNSTGDFDLHLLKVSEGTTILLNCTGQVKGRKPAALGE AQPTKSLEENKSLKEQKKLNDLCFLKRLLQEIKTCWNKILMGTK EH |
| 11 | CCL19 human_aa | MALLLALSLLVLWTSPAPTLSGTNDAEDCCLSVTQKPIPGYIVR NFHYLLIKDGCRVPAVVFTTLRGRQLCAPPDQPWVERIIQRLQ RTSAKMKRRSS |
| 13 | 2A peptide cleavage region | DXEXNPGP |
| 14 | F2A_aa | GSGVKQTLNFDLLKLAGDVESNPGP |

The above were combined to design the #725, #744, #745 and #746 constructs listed in Table 2 below.

**[Table 2]**

| **Plasmid No.** | **Signal sequence** | **TM** | **Generation** |
|---|---|---|---|
| **#725** | IgG SS | CD8a long | 2nd |
| **#744** | IgG SS | CD8a short | |
| **#745** | CD8 SS | CD8a long | |
| **#746** | CD8 SS | CD8a short | |

DNA fragments encoding each of the above constructs were synthesized by conventional methods and inserted into the above pMSGV vector to produce a CAR expression vector.

### <Production of retrovirus into which CAR expressing vector is introduced>

A retrovirus for introducing a gene into a T cell was produced.

First, using a transfection reagent (product name: Lipofectamine 3000 (manufactured by Life Technologies)), each of the above CAR expressing vectors and p-Ampho plasmid (manufactured by Takara Bio Inc.) were transfected into a GP2-293 packaging cell line (manufactured by Takara Bio Inc.) to produce a retrovirus into which the CAR expressing vector was introduced.

Supernatants including retroviruses into which the CAR expressing vectors were introduced, respectively were collected 48 hours after transfection.

As a culture solution for the GP2-293 cell, DMEM including 10% FCS and 1% Penicillin-Streptmycin (manufactured by FUJIFILM Wako Pure Chemical Corporation) was used. As a culture solution for a T cell used in Examples described later, CTS Immune CELLSR (manufactured by Gibco), 1% Penicillin-Streptmycin (manufactured by FUJIFILM Wako Pure Chemical Corporation), 2.5 ug/mL amphotericin B (manufactured by Bristol Myers Squibb Company), and OpTmizer including 2 mM L-glutamine (manufactured by Gibco) was used.

### <Transduction into T cell>

2 × 10⁶ peripheral blood mononuclear cells collected from blood of a healthy donor were cultured together with IL-2 (manufactured by Miltenyi Biotec) on a plate on which an anti-CD3 monoclonal antibody (5 µg/mL) and RetroNectin (registered trademark) (manufactured by Takara Bio Inc., 25 µg/mL) were immobilized for activation of T cells in a 5% CO₂ incubator at 37°C for three days.

On Day 2 after start of the culture, the above-produced supernatant including the retrovirus into which the CAR expressing vector was introduced was added in an amount of 500 µL per well to a surface-untreated 24 well plate coated with 25 µg/mL RetroNectin (manufactured by Takara Bio Inc.) in advance, and centrifuged at 2000 g for two hours to produce a retrovirus-preloaded plate.

Two retrovirus-preloaded plates were produced for each retrovirus. After completion of centrifugation, the plates were washed with 1.5% BSA/PBS and stored at 4°C until use.

On Day 3 of culture, the activated cells were collected from the plate, and a cell suspension was prepared such that the number of cells was 1 × 10⁵ cells/mL. The cell suspension was added to the retrovirus-preloaded plate in an amount of 1 mL per well, and cultured in a 5% CO₂ incubator at 37°C for 24 hours in the presence of IL-2 to perform first retroviral infection.

On the next day (Day 4 of culture), the cell solution of each well was transferred to the second stored retrovirus-preloaded plate, centrifuged at 500 g for one minute, and then cultured at 37°C for four hours to perform second infection.

After culture at 37°C for four hours, 1 mL of the cell suspension of each well was transferred to a new 12 well cell culture plate, diluted four times with a new culture solution including IL-2 (OpTmizer), and cultured in a 37°C in a 5% CO₂ incubator.

The peripheral blood mononuclear cells were cultured until Day 7 counted from start of culture to obtain an anti-EGFRviiiCAR-T cell (hereinafter, also referred to as "CAR-T cell") which is a T cell into which the CAR expressing vector is introduced. CAR-T cells in which the #725, #744, #745, and #746 constructs are introduced are referred to as #725CAR-T cells, #744CAR-T cells, #745CAR-T cells, and #746CAR-T cells, respectively.

The CAR-T cell includes a nucleic acid encoding an exogenous anti-human EGFRviii CAR, a nucleic acid encoding an exogenous IL-7, and a nucleic acid encoding an exogenous CCL 19.

At the same time, as a CAR negative cell control, a "CAR-IL-7-CCL19 non-expressing-T cell" (a non-gene-introduced cell: non-infection or an activated T cell: ActT) that activated peripheral blood mononuclear cells obtained from the same healthy donor by a similar method but was not retrovirally infected was produced.

### <Activated T cells>

T cells before retrovirus infection in the above "Transduction of T cells" were used as "activated T cells" for control.

### <Confirmation of expression of CAR>

### [Flow cytometry analysis]

An expression level of the CAR-T cells was measured by flow cytometry.

The activated T cells and the CAR-T cells were caused to react with antibodies for scFv detection produced by the inventors, a recombinant EGFRviii protein and an allophycocyanin (APC)-labeled anti-CD8 monoclonal antibody (manufactured by Affymetrix, Inc.) to perform staining.

CytoFLEX S (manufactured by Beckman Coulter, Inc.) was used as the flow cytometer, and FlowJo software (manufactured by Tree Star, Inc.) was used for data analysis.

Note that the activated T cells are control.

### (Results)

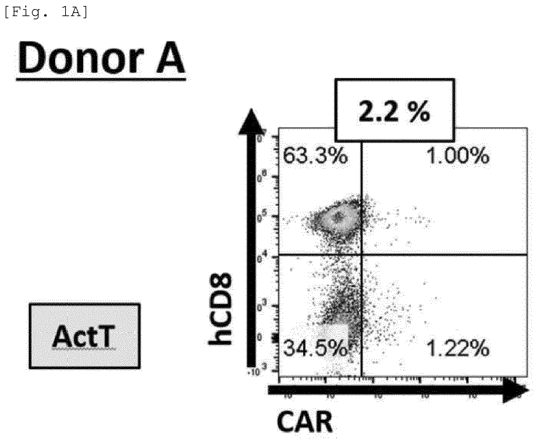

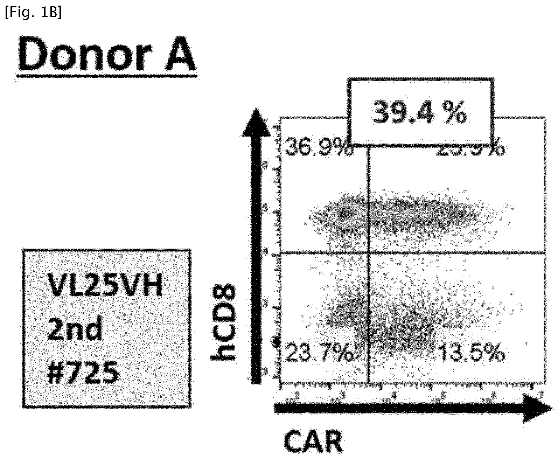

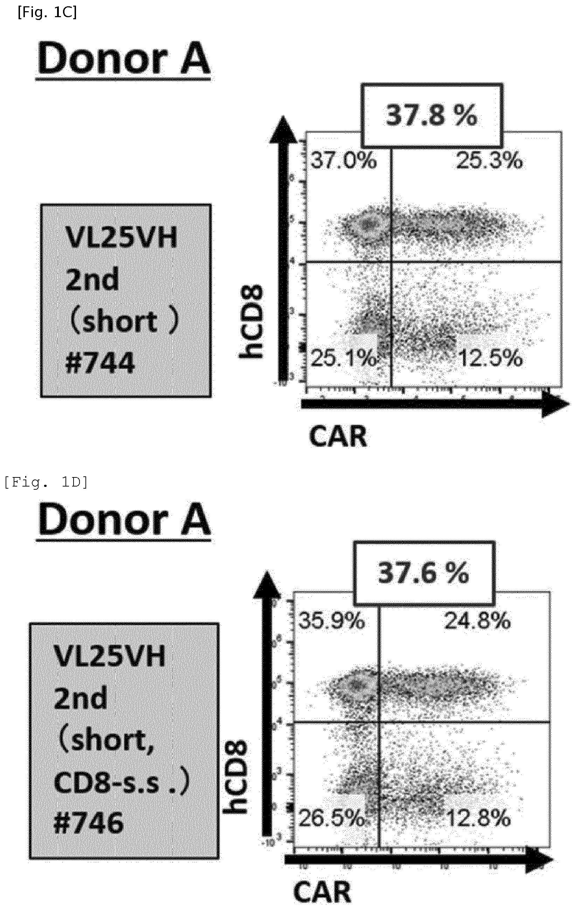

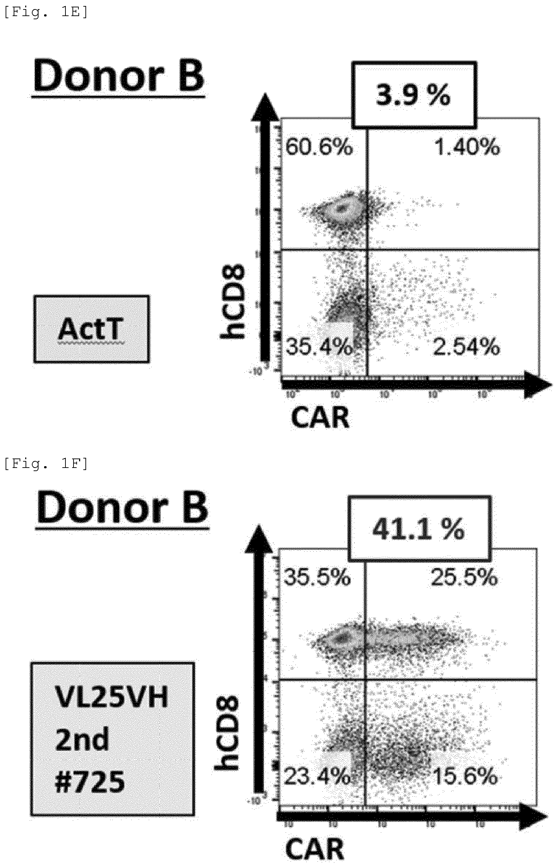

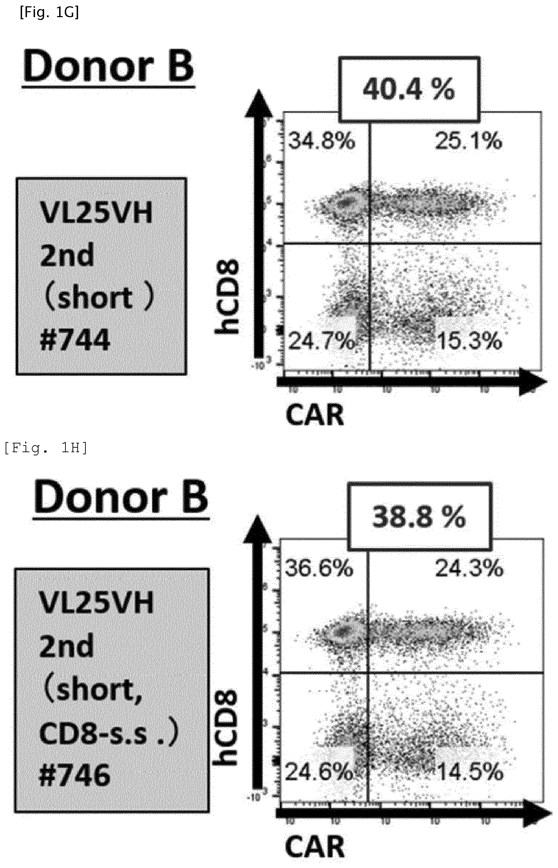

The results are illustrated in Figs. 1A to 1H. In each of Figs. 1A to 1H, the horizontal axis represents expression of CAR, and the vertical axis represents expression of CD8. In the figure, "ActT" represents the control, activated T cell population.

As illustrated in Figures 1A-1H, in both samples from the two donors, a high CAR expression rate of about 40% was observed in the group of cells in which the CAR expression vector was introduced. No significant differences in expression rates were observed even when the signal sequence and transmembrane region sequences were different. In the group of the activated T cell (control: ActT) in which the CAR expression vector was not introduced, CD8 expression was observed, but little CAR expression was observed.

### (Example 2) Evaluation of anti-tumor activity of CAR-T cells (1)

### <Measurement of cytotoxic activity of CAR-T cells targeting tumor cells by ATP assay>

Using #744CAR-T cells and #725CAR-T cells, ATP assays were performed with various ratios of T cells to tumor cells (E:T ratio) to measure cytotoxic activity. The #744CAR-T cells and #725CAR-T cells have the same composition except that they include CD8 Short and CD8 Long as transmembrane regions, respectively.

### -Preparation of tumor cell culture medium-

To 500 mL of a MEM medium, 50 mL of FBS and 5 mL of Penicillin-Streptomycin, Liquid were added to prepare a tumor cell culture medium (also referred to as a tumor cell medium).

### -Co-culture-

Tumor cells (glioblastoma cells U87MGviii 4.12 described later) were diluted to 5 × 10⁵ cell/mL in the tumor cell medium prepared above, and the diluted tumor cells were dispensed into a collagen-coated 96 well plate at 100 µL/well.

Note that the cell number and viability of the PRIME EGFRviiiCAR-T cells were measured with a cell counter.

CAR positive cells were suspended in the tumor cell medium such that the CAR positive cells had a concentration of 5 × 10⁵ cells/mL from data of the cell counter and a CAR positive ratio to prepare a CAR-T cell liquid.

The CAR-T cell liquid was serially diluted with the tumor cell medium and added to the well plate into which the tumor cells were dispensed at 100 µL/well to perform preparation such that the E : T ratio was 0 : 1, 0.01 : 1, 0.03 : 1, 0.1 : 1, 0.3 : 1, or 1 : 1.

The above 96-well plate was placed in a CO₂ incubator set at 37°C and 5 vol% CO₂ concentration and co-cultured for 48 hours.

The tumor cell culture medium was removed from the well plate taken out of the CO₂ incubator and washed with PBS. Frozen stored CellTiter-Gro Reagent (manufactured by Promega Corporation) was thawed in a light-shielded manner. Thereafter, the whole amount of one vial of CellTiter-Gro substrate (manufactured by Promega Corporation) was put into 10 mL of CellTiter-Gro buffer (manufactured by Promega Corporation), mixed, and dispensed into a 96 well plate at 100 µL/well.

A lid of the 96 well plate was covered with aluminum foil, and the 96 well plate was shaken at 25°C and 200 revolutions per minute (rpm) for 10 minutes to cause a reaction with ATP. Immediately after the reaction, luminescence was measured (Shake 5 sec (60 rpm), Delay 30 sec) with a plate reader, and the number of living cells was measured.

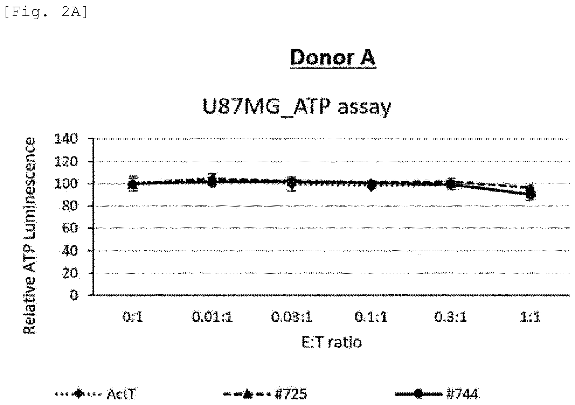

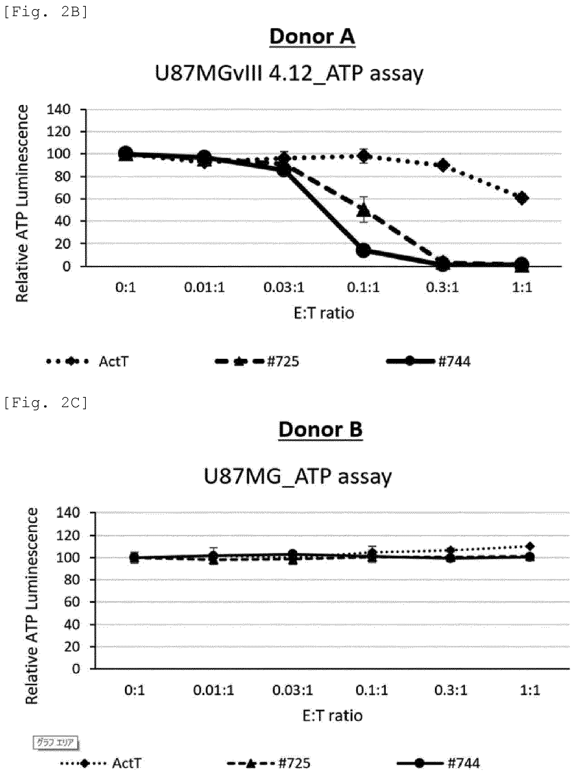

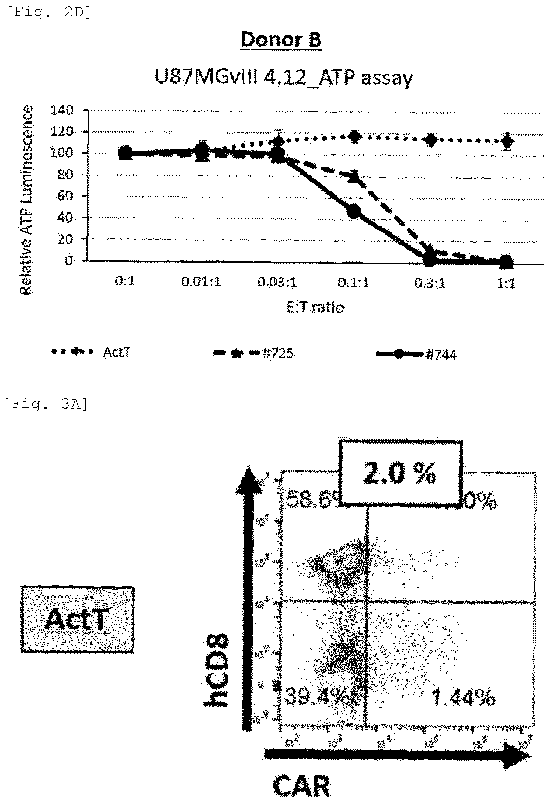

As a control, glioblastoma cell line U87MG cells not expressing EGFRviii were used in place of U87MGviii cells for measurement in a similar manner. The results are illustrated in Figs. 2A to 2D.

Figures 2A-2D show the decay rate of luminescence at each E:T ratio expressed as a percentage (%) when the numerical value of ATP luminescence intensity of the 96-well plate to which only tumor cells were added is 100%. The decay rate of luminescence indicates tumor cell viability.

As illustrated in Figures 2A-2D, CAR-T cells exhibited cytotoxic activity specific to EGFRviii expressing tumor cells in both samples derived from the two donors. The cytotoxic activity of #744 CAR-T cells with CD8 Short in the transmembrane region was higher than that of #725 CAR-T cells with CD8 Long. The activated T cell group showed little cytotoxic activity, regardless of the expression of EGFRviii by the tumor cells.

### (Example 3) Production of CAR-T cells (2)

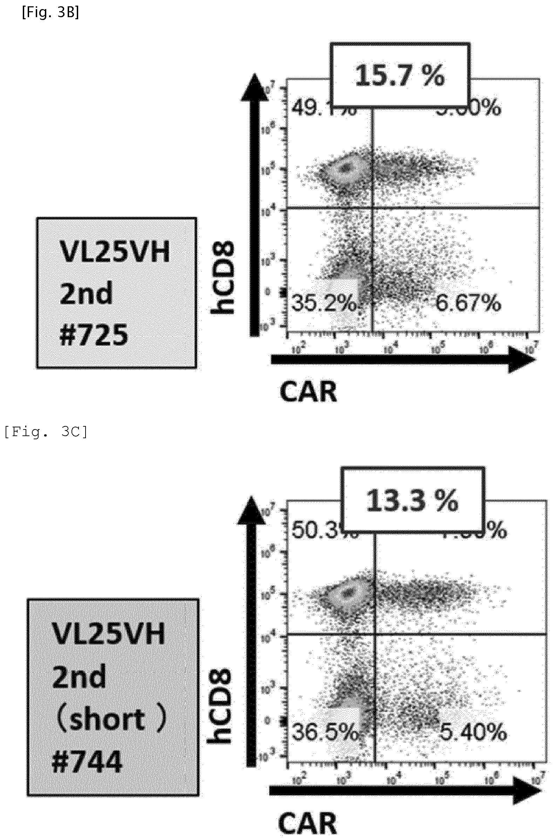

From another peripheral blood mononuclear cell sample, #725CAR-T cells and #744CAR-T cells were produced by the same method as in Example 1, and the expression rate of CAR was measured. The results are shown in Figures 3a-3c.

Sufficient CAR expression was observed in both #725CAR-T cells and #744CAR-T cells. No significant differences in expression rates were observed even when the sequence of the transmembrane region differed.

### (Example 4) Evaluation of anti-tumor activity of CAR-T cells (2)

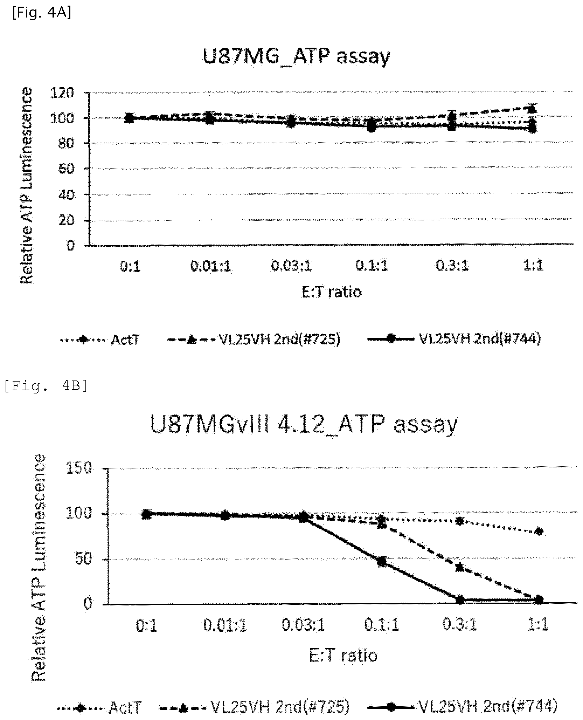

Using the CAR-T cells produced in Example 3, the cytotoxic activity of the CAR-T cells was evaluated in the same manner as in Example 2. The results are shown in Figures 4A and 4B.

As in Example 2, CAR-T cells showed cytotoxic activity specific for EGFRviii expression. The cytotoxic activity of #744 CAR-T cells with CD8 Short in the transmembrane region was higher than that of #725 CAR-T cells with CD8 Long. The activated T cell group showed little cytotoxic activity, regardless of the expression of EGFRviii by tumor cells.

### (Example 5) Evaluation of in vivo anti-tumor activity of CAR-T cells

In vivo anti-tumor activity was evaluated for #744 CAR-T cells using CD8 Short in the transmembrane region.

### <Confirmation of expression of EGFRviii in glioblastoma cells U87MGviii 4.12>

### [Flow cytometry analysis]

Expression of EGFRviii in glioblastoma cells U87MGviii 4.12 (hereinafter, also simply referred to as "tumor cells") was confirmed for use in an assay to study cytotoxic activity and in preparing an animal model to study anti-tumor activity in vivo.

Specifically, a hybridoma culture supernatant of an anti-human EGFRviii antibody (clone 1B10-1A12) was added at 50 uL/sample, then an anti-mouse IgG antibody (Cat. No. 405308, manufactured by BioLegend, Inc.) labeled with allophycocyanin (APC) as a secondary antibody was stained at 50 ng/sample, and flow cytometry analysis was performed.

CytoFLEX S (manufactured by Beckman Coulter, Inc.) was used as the flow cytometer, and FlowJo software (manufactured by Tree Star, Inc.) was used for data analysis.

### (Results)

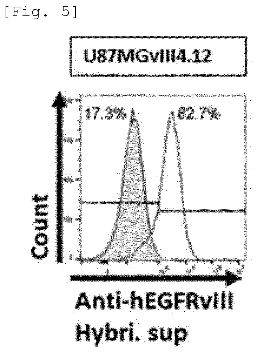

The results are illustrated in Fig. 5. 80% or more of tumor cells were confirmed to express EGFRviii.

### <Therapeutic effect using mouse tumor model>

A double-deficient mouse (NOG-lab/b2m dKO mouse) in which an NGO gene and a B2M gene were deficient was inoculated with 1.0 × 10⁶ glioblastoma cells U87MGviii 4.12, which were confirmed to express EGFRviii, subcutaneously to prepare a tumor model mouse.

Ten days after subcutaneous inoculation, to five tumor model mice in each group, a Hank's balanced salt solution (HBSS) was administered intravenously as an untreated group, 1.25 × 10⁷ activated T cells were administered intravenously as an activated T cell administration group, and 1 × 10⁷ #744 CAR-T cells (1.25 × 10⁷ cells from a cell group having a CAR expression ratio of 80%) were administered intravenously as a EGFRviii CAR-T cell administration group.

Thereafter, a tumor volume and a body weight of each of the model mice were measured twice a week, and a model mouse which decreased the body weight by 20% or a model mouse which was significantly weakened was dissected to confirm an anatomical photograph and presence or absence of tumor metastasis.

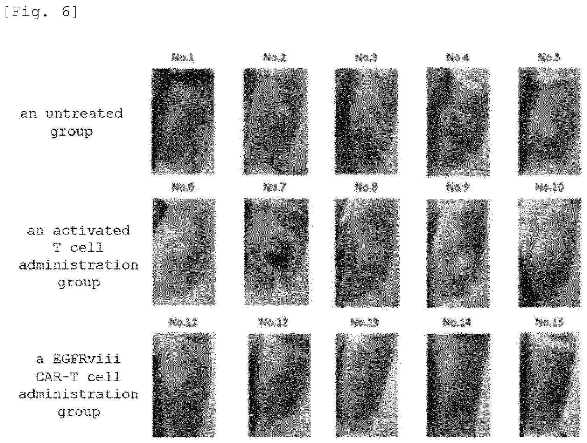

Photographs of backs of the tumor model mice inoculated with tumor cells are illustrated in Fig. 6.

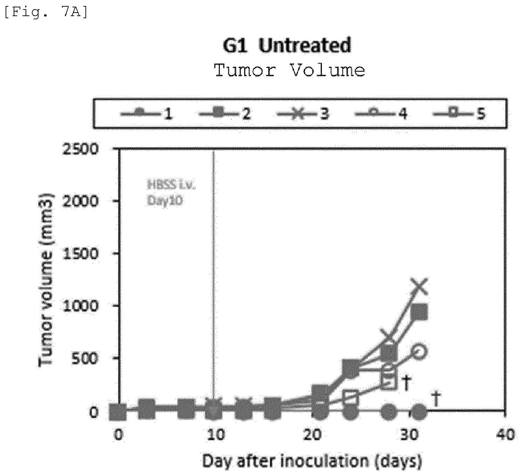

Results of changes in tumor volumes of the tumor model mice are illustrated in Figs. 7A to 7C. The horizontal axis represents the number of days after inoculation of tumor cells into mice (the day of inoculation into mice was day 0), and the vertical axis represents a tumor volume (major axis of tumor × (minor axis of tumor)²/2 (mm³)). The number of each piece of data is an individual identification number.

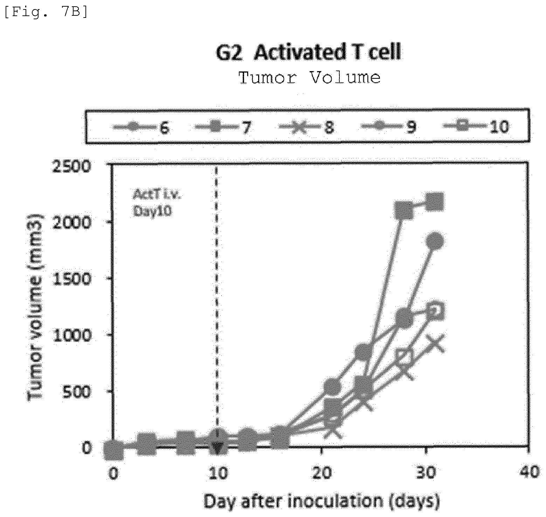

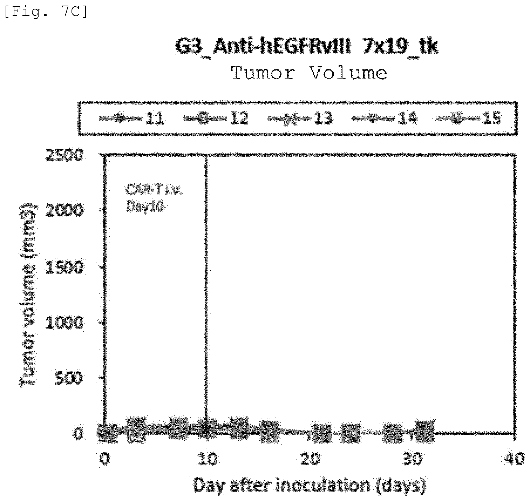

As illustrated in Figures 7A-7C, in the untreated group (Figure 7A), the tumor volume increased except for one case in which tumor cells were not engrafted. In the activated T cell administration group (Figure 7B), a significant increase in tumor volume was observed in each case. Meanwhile, in the PRIME EGFRviii CAR-T cell administration group (Figure 7C), the tumor volume hardly increased.

As illustrated in Figs. 11 and 12A to 12C, it was confirmed that PRIME EGFRviii CAR-T cells (anti-EGFRviiiCAR-IL-7/CCL19-expressing T cells) exhibited excellent anti-tumor activity in tumor model mice.

As illustrated in Figs. 6 and 7A to 7C, it was confirmed that #744 CAR-T cells exhibited excellent antitumor activity in tumor model mice.

## Claims

1. A chimeric antigen receptor includes a target antigen binding region, a transmembrane region, and a signal transduction region, wherein the transmembrane region includes at its C-terminus a first polypeptide selected from the group consisting of (1a) to (1c) below.
(1a) a polypeptide consisting of an amino acid sequence in which at least 3 amino acids from the C-terminus are deleted in the amino acid sequence set forth in SEQ ID NO: 1;
(1b) a polypeptide consisting of an amino acid sequence having 85% or more sequence identity to the amino acid sequence of the polypeptide of (1a); and
(1c) a polypeptide consisting of an amino acid sequence in which one or more amino acids are mutated in the amino acid sequence of the polypeptide of (1a).

2. The chimeric antigen receptor according to claim 1, wherein the polypeptide of (1a) is a polypeptide consisting of an amino acid sequence in which 7 amino acids from the C-terminus of the amino acid sequence set forth in SEQ ID NO: 1 are deleted.

3. The chimeric antigen receptor according to claim 1 or 2, wherein the transmembrane region further includes a second polypeptide selected from the group consisting of the following (2a) to (2c) on the N-terminal side of the first polypeptide:
(2a) a polypeptide consisting of an amino acid sequence in which at least three amino acids from the N-terminus are deleted in the amino acid sequence set forth in SEQ ID NO: 2;
(2b) a polypeptide consisting of an amino acid sequence having 85% or more sequence identity to the amino acid sequence of the polypeptide of (2a); and
(2c) a polypeptide consisting of an amino acid sequence in which one or more amino acids in the amino acid sequence of the polypeptide of (2a) are mutated.

4. The chimeric antigen receptor according to claim 3, wherein the polypeptide of (2a) is a polypeptide consisting of an amino acid sequence in which 10 amino acids from the N-terminal of the amino acid sequence set forth in SEQ ID NO: 2 are deleted.

5. The chimeric antigen receptor according to any one of claims 1 to 4, wherein the signal transduction region is a T cell activation signal transduction region.

6. A cell expressing the chimeric antigen receptor according to any one of claims 1 to 5.

7. The cell according to claim 6, further expressing at least one of IL-7 and CCL19.

8. The cell according to claim 6 or 7, wherein the cell is an immune cell.

9. The cell according to claim 8, wherein the immune cell is a T cell.

10. A polynucleotide including a nucleotide sequence encoding the chimeric antigen receptor according to any one of claims 1 to 5.

11. A polynucleotide according to claim 10, wherein the chimeric antigen receptor further including a signal peptide at the N-terminus, the signal peptide is a polypeptide selected from the group consisting of the following (3a) to (3f):
(3a) a polypeptide consisting of an amino acid sequence as described in SEQ ID NO: 3;
(3b) a polypeptide consisting of an amino acid sequence having 85% or more sequence identity with the amino acid sequence set forth in SEQ ID NO: 3; and
(3c) a polypeptide consisting of an amino acid sequence in which one or more amino acids in the amino acid sequence set forth in SEQ ID NO: 3 are mutated;
(3d) a polypeptide consisting of the amino acid sequence set forth in SEQ ID NO: 4;
(3e) a polypeptide consisting of an amino acid sequence having 85% or more sequence identity with the amino acid sequence set forth in SEQ ID NO: 4; and
(3f) a polypeptide consisting of an amino acid sequence in which one or more amino acids in the amino acid sequence set forth in SEQ ID NO: 4 are mutated.

12. The polynucleotide according to claim 11, further including at least one of a nucleotide sequence encoding IL-7 and a nucleotide sequence encoding CCL19.

13. A vector including the polynucleotide of claim 11 or 12.

14. A method for producing a cell expressing a chimeric antigen receptor, the method including introducing the vector according to claim 13 into a cell.

15. A pharmaceutical composition including the cell according to any one of claims 6 to 9.

16. The pharmaceutical composition according to claim 15, which is a pharmaceutical composition for treating or preventing a tumor.
